# FASCICULE DE BREVET EUROPEEN

(11) **EP 0 516 530 B1**
(45) Date de publication et mention de la délivrance du brevet: **21.08.1996**
(21) Numéro de dépôt: 92401439.2
(22) Date de dépôt: 26.05.1992
(51) Int. Cl.: C07J 31/00, C07J 71/00, A61K 31/565

(54) **Nouveaux produits stéroides substitués en positions 6 et comportant, en position 10, un radical thioéthyle, leur procédé de préparation et les intermédiaires de ce procédé, leur application comme médicaments et les compositions pharmaceutiques les renfermant**
Neue 6-substituierte Steroide mit einem Thioethyl-Substituent an der 10-Position, ein Verfahren zu ihrer Herstellung sowie Zwischenprodukte dafür, ihre Verwendung als Arzneimittel und pharmazeutische Präparate davon
New 6-substituted steroids with a thioethyl group in the 10 position, a process for their preparation and intermediates therefor, their use as medicines and pharmaceutical compositions thereof

(30) Priorité: 27.05.1991 FR 9106332
(43) Date de publication de la demande: 02.12.1992
(73) Titulaire: ROUSSEL UCLAF, 93230 Romainville (FR)
(72) Inventeur: Gourvest, Jean-François, F-77410 Claye-Souilly (FR); Lesuisse, Dominique, F-75018 Paris (FR)
(74) Mandataire: Vieillefosse, Jean-Claude

(56) Documents cités:
- EP-A- 0 149 499
- EP-A- 0 434 570
- GB-A- 2 078 749
- US-A- 4 087 524
- US-A- 4 139 617
- BIOCHEMISTRY, vol. 26, no. 24, 01 décembre 1987, Easton, PA (US); D.D. BEUSEN et al., pp. 783-7841

## Description

La présente invention concerne de nouveaux produits stéroïdes substitués en position 6 et comportant, en position 10, un radical thioéthyle, leur procédé de préparation et les intermédiaires de ce procédé, leur application comme médicaments et les compositions pharmaceutiques les renfermant.

Dans les documents EP-A-149 499 et GB-A-2 078 749 sont décrits des stéroides 10 substitués ayant une activité antiaromatase.

Dans la demande de brevet EP-A-149 499, les composés décrits diffèrent des produits de la présente demande en ce qu'il ne comportent pas de substitution en position 6 et en ce que la liaison 9-11 est saturée.

Dans la demande de brevet GB-A-2 078 749 les composés décrits diffèrent des produits de la présente demande en ce qu'il comportent en position 10 un groupement alkynyle et en ce que la liaison 9-11 est saturée.

L'invention a pour objet les produits de formule générale (I) : 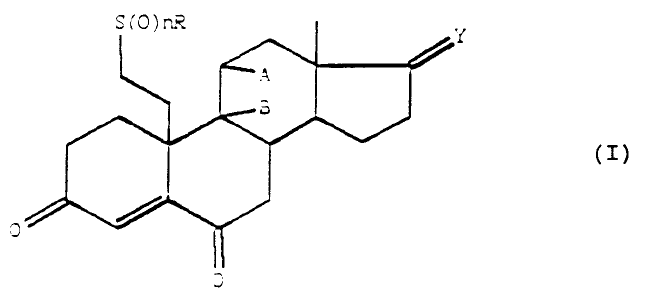 dans laquelle le substituant R représente un radical alkyle, alcényle ou alcynyle ayant au plus 4 atomes de carbone, Y représente un atome d'oxygène ou un reste : 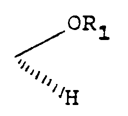 dans lequel R₁ représente un atome d'hydrogène ou un radical acyle, n représente un entier de 0 à 2, D représente un atome d'oxygène ou un reste 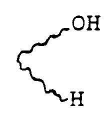
A et B forment ensemble une fonction époxyde alpha ou avec les atomes de carbone auxquels ils sont liés, une seconde liaison entre ces carbones.

Parmi les radicaux alkyle, on peut citer les radicaux méthyle, éthyle, propyle, isopropyle, butyle, isobutyle, butyle secondaire, tert-butyle.

Parmi les radicaux alcényle et alcynyle, on peut citer les radicaux vinyle, allyle, 1-propényle, éthynyle, 1 ou 2-propynyle.

Parmi les radicaux acyles, on préfère le reste d'un acide aliphatique ou cycloaliphatique saturé ou insaturé et notamment le reste d'un acide alcanoïque tel que par exemple l'acide acétique, propionique, butyrique ou isobutyrique, valérique ou undécylique, le reste d'un acide hydroxyalcanoïque tel que par exemple l'acide hydroxyacétique, le reste d'un acide cycloalkylcarboxylique ou (cycloalkyle) alcanoïque tel que par exemple l'acide cyclopropyl, cyclopentyl ou cyclohexylcarboxylique, cyclopentyle ou cyclohexyle acétique ou propionique, le reste d'un acide benzoïque ou d'un acide phénylalcanoïque tel que l'acide phényl acétique ou phényl propionique, le reste d'un amino acide tel que l'acide diéthylamino acétique ou aspartique ou le reste de l'acide formique. On préfère les radicaux acétyle, propionyle ou benzoyle.

Parmi les valeurs de R on préfère les radicaux méthyle ou éthyle.

Parmi les valeurs de R_{**1**} on préfère les radicaux hydrogène ou acétyle.

L'invention a plus particulièrement pour objet les produits de formule générale (I′) : 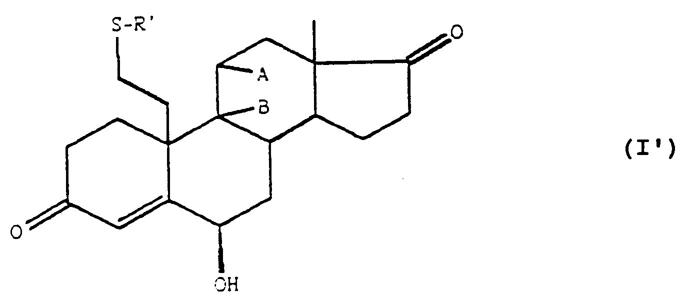
dans laquelle A et B ont la signification précédente et R′ représente un radical alkyle ayant au plus 4 atomes de carbone.

Parmi les produits de formule (I′) telle que définie ci-dessus, on préfère les produits dans lesquels R′ est choisi parmi les radicaux méthyle ou éthyle, en particulier méthyle.

Parmi les produits de formule (I′), l'invention a particulièrement pour objet les produits décrits ci-après dans les exemples, en particulier :
- 6béta-hydroxy 10béta-[2-(méthylthio) éthyl] estra-4,9(11)-diène-3,17-dione,
- 9alpha,11alpha-époxy 6béta-hydroxy 10béta-[2-(méthylthio) éthyl] estr-4-ène-3,17-dione.

La présente invention a également pour objet un procédé de préparation des produits de formule générale (I) telle que définie ci-dessus et caractérisé en ce que l'on soumet un produit de formule (II) : 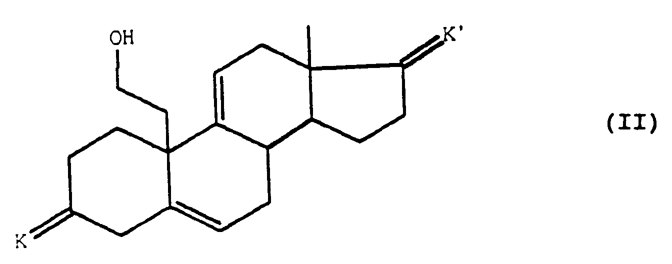
dans laquelle K et K′, identiques ou différents représentent un radical céto protégé, à l'action d'un réactif d'époxydation pour obtenir les produits de formule (IIIa) et (IIIb) : 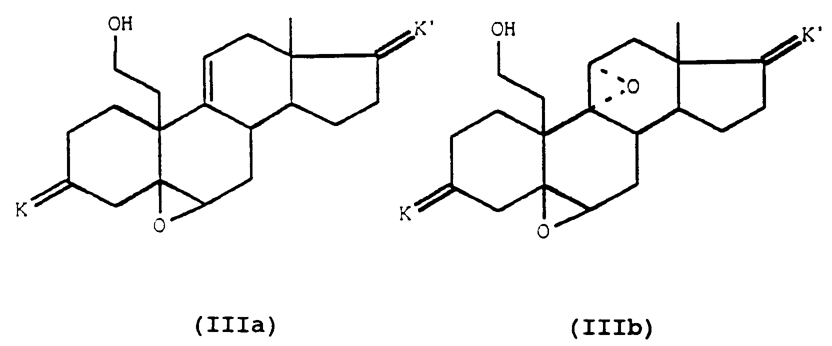
produits que l'on traite :
ou bien par un réactif de transformation du radical hydroxy en radical thiol pour obtenir des produits de formules (IVa) et (IVb) : 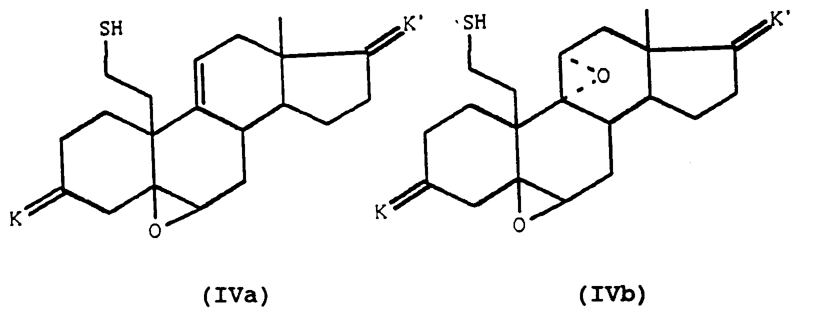
que l'on traite par un dérivé réactif du radical R pour obtenir les produits de formules (Va) et (Vb) : 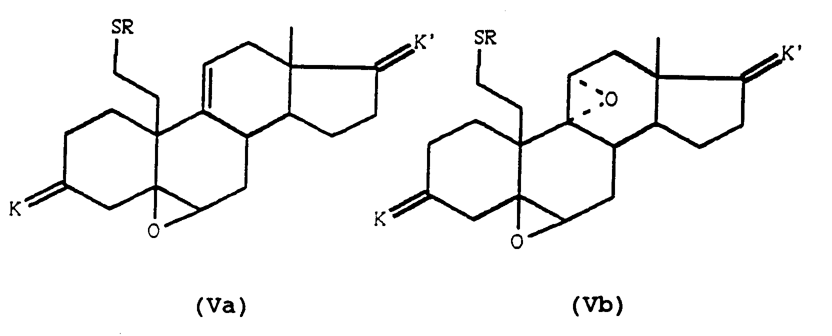
ou bien par un dérivé réactif d'un acide sulfonique pour obtenir les produits de formules (VIa) et (VIb) : 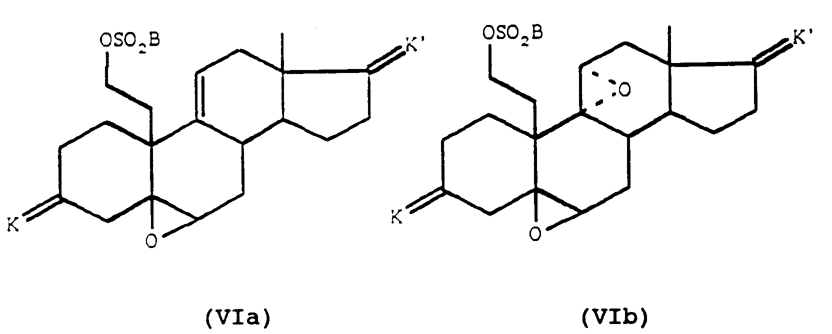
dans laquelle B représente le reste d'un acide sulfonique produits de formules (VIa) et (VIb) que l'on traite par un sel de formule RSA dans laquelle A représente un cation monovalent pour obtenir des produits de formules (Va) et (Vb) telles que définies ci-dessus, produits de formules (Va) et (Vb) que l'on traite par un réactif de déprotection des fonctions cétones protégées et d'ouverture de l'époxyde en position 5(6) pour obtenir des produits de formules (I′a) et (I′b) : 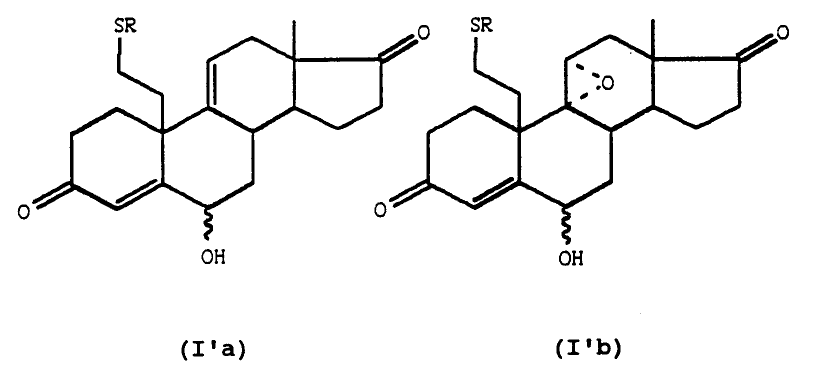
correspondant aux produits de formule (I) dans laquelle R a les significations indiquées précédemment, Y représente un atome d'oxygène, n représente le nombre 0, et D représente un reste : 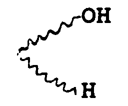
produits de formule (I′a) et (I′b) que, si désiré, l'on soumet à l'une quelconque des réactions suivantes :
a) réduction suivie éventuellement d'une acylation de la fonction cétone en position 17,
b) oxydation de l'atome de soufre du radical -SR en sulfoxyde ou en sulfone,
c) oxydation de la fonction hydroxyle en position 6 en cétone.

Dans les conditions préférentielles d'exécution du procédé décrit ci-dessus, on opère comme suit :
- le réactif d'époxydation que l'on utilise pour transformer les produits de formule (II) en produits de formule (III) est l'acide 3-chloroperbenzoïque mais on peut également utiliser d'autres peracides tels que l'acide monoperphtalique ou son sel de magnésium ou encore l'eau oxygénée en présence d'hexachloro (ou hexafluoro) acétone, ou encore le peroxymonosulfate de potassium (oxone),
- le réactif de transformation du radical hydroxyle en radical thiol est l'azodicarboxylate de diéthyle en présence de tri-phénylphosphine et d'acide thioacétique ; on obtient ainsi intermédiairement un produit comportant en position 10béta un radical acétylthioéthyle qui est transformé en radical thiol par action de l'hydrazine. Les deux stades de la réaction sont effectués de préférence dans un solvant aprotique tel que le tétrahydrofuranne ou l'éther diéthylique,
- le dérivé réactif du radical R que l'on utilise de préférence est un halogénure tel que le chlorure ou le bromure, on peut également utiliser un pseudohalogénure tel qu'un mésylate ou un tosylate. On opère en présence d'une base forte telle qu'un alcoolate de métal alcalin par exemple le tert-butylate de potassium, ou un amidure par exemple l'amidure de diisopropyl lithium, ou l'hexaméthyldisilylazanate de lithium ou de potassium. On peut opérer dans un solvant tel que le tétrahydrofuranne à basse température (par exemple entre 0 et -78°C),
- le dérivé réactif de l'acide sulfonique de formule BSO_{**3**}H dans laquelle B représente de préférence un radical méthyle ou tolyle est de préférence un halogénure tel que le chlorure. On utilise de préférence le chlorure de mésyle. On opère de préférence en présence d'une base minérale ou organique de préférence la triéthylamine. On utilise un solvant de réaction tel que le chlorure de méthylène et la réaction est conduite à une température de l'ordre de 0°C,
- le sel de formule RSA dans laquelle R a la signification précédente, est de préférence un sel de métal alcalin tel que le sodium ou le lithium. L'action de ce sel est effectuée de préférence dans un solvant aprotique tel que le tétrahydrofuranne, l'hexaméthylphosphoramide ou le diméthylformamide ; on peut travailler en présence d'un éther couronne spécifique du métal utilisé comme par exemple le 12-crown-4 pour le lithium, le 15-crown-5 pour le sodium, ou le 18-crown-6 pour le potassium. L'action du sel de formule RSA sur les produits de formules (VIa) et (VIb) peut conduire à un déblocage partiel ou total de l'une ou des deux fonctions cétoniques protégées en positions 3 et 17. On peut notamment obtenir des produits de formules (V′a) et (V′b) : 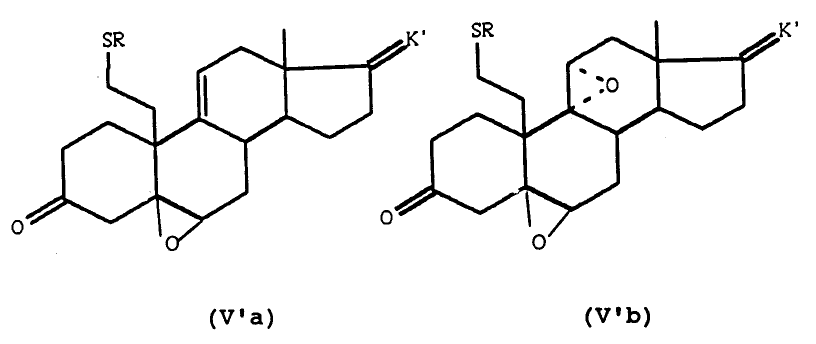 qui peuvent, bien entendu conduire aux produits de formules (I′a) et (I′b) dans les mêmes conditions que les produits de formules (Va) et (Vb),
- l'hydrolyse des produits de formules (Va) et (Vb) en produits de formules (I′a) et (I′b) est de préférence une hydrolyse acide par exemple à l'aide d'acide chlorhydrique dilué (2 à 6N) ou d'acide acétique dans un solvant tel que l'éthanol ou le tétrahydrofuranne,
- la réduction de la fonction cétone en position 17 est effectuée à l'aide d'un hydrure tel que le borohydrure de sodium dans un solvant tel que le méthanol.
- l'acylation éventuelle de cette fonction cétone en position 17 est effectuée par action du dérivé réactif du radical acyle à introduire. On peut ainsi utiliser un halogénure d'acide notamment un chlorure d'acide, un anhydride mixte ou symétrique. On peut citer par exemple le chlorure d'acétyle ou l'anhydride acétique,
- l'oxydation éventuelle de l'atome de soufre en sulfoxyde est effectuée par exemple par action d'un périodate tel que le périodate de sodium dans un solvant aqueux tel que le méthanol aqueux ou d'un peracide tel que l'acide métachloroperbenzoïque dans un solvant tel que le dichlorométhane,
- l'oxydation éventuelle de cet atome de soufre en sulfone est effectuée à l'aide d'un peracide tel que l'acide métachloroperbenzoïque,
- l'oxydation de la fonction hydroxyle en position 6 en cétone est effectuée par exemple à l'aide de chlorure d'oxalyle diméthyl sulfoxyde (réatif de Swern), de triisopropoxyde d'aluminium en présence de cyclohexanone.

Les radicaux céto protégés que représentent les radicaux K et K′ sont choisis de préférence parmi les diéthers cycliques ou non cycliques tels que les radicaux éthylènedioxy, diméthylpropylènedioxy, diméthoxy ou diéthoxy.

L'invention a plus particulièrement pour objet un procédé selon le procédé indiqué ci-dessus caractérisé en ce que la transformation des produits de formules (Va) et (Vb) est effectuée dans des conditions ménagées pour obtenir les produits de formules (VIIa) et (VIIb) : 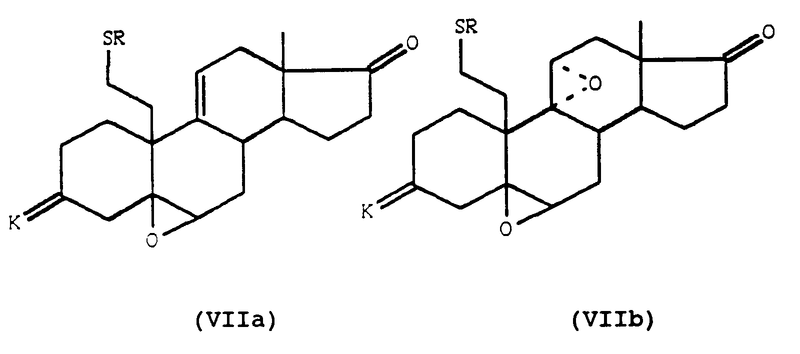
produits de formules (VIIa) et (VIIb) que l'on traite par un réactif de déprotection de la fonction cétone et d'ouverture de l'époxyde en position 5(6) pour obtenir les produits de formules (I′a) et (I′b) telles que définies ci-dessus.

Les conditions ménagées dans lesquelles la tranformation des produits de formules (Va) et (Vb) en produits de formules (VIIa) et (VIIb) est effectuée consistent à utiliser par exemple de l'acide chlorhydrique plus dilué que celui indiqué ci-dessus par exemple de l'acide chlorhydrique normal dans un solvant tel que l'éthanol.

La transformation ultérieure des produits de formules (VIIa) et (VIIb) en produits de formules (I′a) et (I′b) est effectuée dans les mêmes conditions par exemple à l'aide d'acide chlorhydrique normal dans l'éthanol.

L'observation selon laquelle environ 35 % des cancers du sein sont estrogéno-dépendants a conduit à rechercher les moyens de limiter la production d'estrogènes.

Après avoir utilisé des méthodes chirurgicales consistant à supprimer les sources d'estrogènes (ovaires) ou les sources de leurs précurseurs biosynthétiques, les androgènes (glandes surrénales) on a cherché à développer des méthodes moins traumatisantes.
(ABUL-HAJJ Y.J.O. Steroid Biochem 13 (1980), 1935 ; BRODIE A.M.H. Cancer Res. 42, (1982), 3312).

A cet égard, l'inhibition spécifique du dernier stade enzymatique de l'aromatisation des androgènes 3-céto delta-4 en estrogènes phénoliques, semble le moyen le plus efficace et le moins perturbant. L'enzyme responsable de cette transformation est une mono-oxygénase connue comme étant un cytochrome P450 : l'AROMATASE (BRODIE A.M.H. J. Endocrinol. Invest. 2 (1979) 445) qui requiert de l'oxygène et la NADPH (Nicotinamide Adenine Dinucleotide phosphate réduit) pour effectuer l'aromatisation des androgènes en estrogènes.

Sur la base d'un autre mécanisme, d'autres auteurs (par exemple MARCOTTE et Al, Biochemistry 21, (1982) 2773, FLYNN et Al Biochem. Biophys. Res. Com. 103 (1981) 713) ont proposé des inhibiteurs suicides pour l'Aromatase. Des inhibiteurs compétitifs tels que l'Aminoglutéthimide ont également été proposés dans le traitement des cancers métastasiques du sein. Ce produit s'est cependant révélé comme n'étant pas spécifique de l'Aromatase : il s'attaque en effet à d'autres processus enzymatiques que celui conduisant des androgènes aux estrogènes.

Les produits objets de la présente invention présentent au contraire une activité spécifique de l'aromatase (cytochrome P450 aromatase).

Cette propriété inhibitrice de l'aromatase rend les produits de la présente invention aptes à être utilisés dans les pathologies hormono-dépendantes, plus particulièrement estrogéno-dépendantes, comme par exemple :
- cancers du sein, de l'endomètre, de l'ovaire et du pancréas,
- gynécomasties,
- troubles bénins du sein,
- endométriose,
- affections polycystiques de l'ovaire,
- hyperplasie prostatique et plus généralement dans le traitement des hyperestrogènémies,
- certaines formes d'obésité.

L'invention a donc pour objet, à titre de médicaments, les produits de formule générale (I) telle que décrite ci-dessus.

L'invention a plus spécialement pour objet, à titre de médicaments, les produits de formule générale (I′) et plus particulièrement ceux dans laquelle R′ est choisi parmi les radicaux méthyle et éthyle.

L'invention a tout spécialement pour objet, à titre de médicaments :
- 6béta-hydroxy 10béta-[2-(méthylthio) éthyl] estra-4,9(11)-diène-3,17-dione,
- 9alpha,11alpha-époxy 6béta-hydroxy 10béta-[2-(méthylthio) éthyl] estr-4-ène-3,17-dione.

La posologie varie en fonction de l'affection à traiter et de la voie d'administration. Elle peut varier par exemple de 0,1 à 50 mg/kg de préfèrence 0,5 à 10 mg/kg et par jour chez l'adulte par voie orale.

Les nouveaux produits de formule (I) peuvent être employés pour préparer des compositions pharmaceutiques renfermant, à titre de principe actif, l'un au moins desdits produits.

Les produits de formule (I) sont utilisés par voie digestive, parentérale ou locale par exemple par voie transdermique. Ils peuvent être prescrits sous forme de comprimés simples ou dragéifiés, de gélules, de granulés, de suppositoires, d'ovules, de préparations injectables, de pommades, de crèmes, de gels, de patches, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a donc pour objet les compositions pharmaceutiques renfermant comme principe actif au moins un produit de formule (I) et plus particulièrement un produit de formule (I′).

L'invention a également pour objet à titre de produits industriels nouveaux et notamment à titre de produits industriels nouveaux nécessaires pour la préparation des produits de formule (I), les produits de formules générales (IIIa), (IIIb), (IVa), (IVb), (Va) et (VIb) ainsi que les produits de formules (VIIa) et (VIIb), telles que définies ci-dessus.

Les produits de formule (II) utilisés au départ du procédé de l'invention peuvent être préparés comme suit :
On effectue sur le produit de formule (A) : 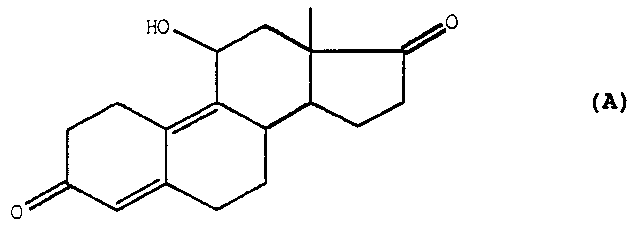
un réarrangement de Claisen pour obtenir un produit de formule (B) : 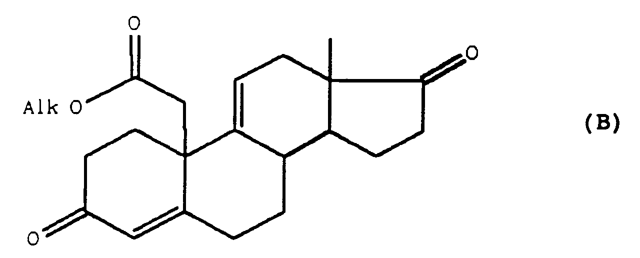
dans laquelle Alk représente un radical alkyle ayant de 1 à 4 atomes de carbone, et soumet le produit de formule (B) à un ou deux réactifs de protection du radical cétone pour obtenir un produit de formule (C) : 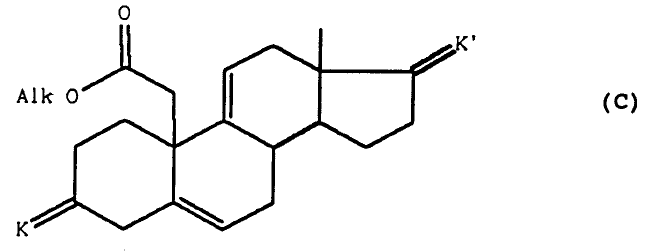
que l'on soumet à une réduction pour obtenir les produits de formule (II).

Dans un mode préférentiel d'exécution de ce procédé le réarrangement de Claisen est effectué à l'aide d'un ortho-acétate d'alkyle, de préférence d'éthyle, en présence par exemple d'un acide organique tel que l'acide propionique et éventuellement dans un solvant tel que le xylène. La réaction s'effectue de préférence à température élevée.

Le réactif de protection des fonctions cétones est choisi parmi les alcools ou les diols. On utilise de préférence l'éthylèneglycol. La réaction s'effectue de préférence en présence d'un acide tel que l'acide paratoluènesulfonique. La réaction s'effectue en chauffant, par exemple au reflux d'un solvant tel que le dichloroéthane, ou en utilisant l'éthylène-glycol lui-même comme solvant.
- la réduction à laquelle on soumet les produits de formule (C) pour obtenir les produits de formule (II) est effectuée à l'aide d'un hydrure de préférence l'hydrure de lithium aluminium. On opère dans un solvant aprotique tel que le tétrahydrofuranne ou l'éther éthylique, par exemple à température ambiante.

Dans les produits de formules (B) et (C), Alk représente de préférence un radical éthyle.

Le produit de formule (A) est décrit par exemple dans le brevet américain USP 3.282.785.

Par l'expression "12-crown-4" on entend le 1,4,7,10-tétraoxacyclododécane ; "15-crown-5" représente le 1,4,7,10,13-pentaoxacyclopentadécane ; "18-crown-6" représente le 1,4,7,10,13,16-hexaoxacyclooctadécane.

Les exemples suivants illustrent l'invention sans toutefois la limiter.

### Préparation 1 :

Le 3,17-bis(éthylènedioxy) androsta-5,9(11)-diène-19-carboxylate d'éthyle utilisé au départ de l'exemple 1 a été préparé comme suit :

### Stade 1 : 3,17-dioxo androsta-4,9(11)-diène-19-carboxylate d'éthyle.

On amène à une température de 137°C un mélange de 500 mg de 11béta-hydroxy androsta-4,9-diène-3,17-dione (produit décrit dans le brevet américain USP 3.282.785), 5 cm^{**3**} d'ortho-acétate de triéthyle et 6,4 mg d'acide propionique. Après 4 heures de chauffage, le milieu réactionnel est concentré à sec et le résidu est chromatographié sur silice (éluant : acétate d'éthyle-hexane 1-1). On obtient le produit attendu (503 mg, Rf = 0,33).
RMN (CDCl_{**3**}, 250 MHz) 0,94 (s, 18 Me) ; 1,23 (t, COOCH_{**2**}CH_{**3**}) 3,94 à 4,29 (m, COOCH_{**2**}CH_{**3**}), 5,61 (m, H11), 5,84 (large s, H4).
IR (CHCL_{**3**}) 1732 cm^{**-1**} (17-cétone), 1662, 1612 (cétone conjuguée).

### Stade 2 : 3,17-bis(éthylènedioxy) androsta-5,9(11)-diène-19-carboxylate d'éthyle (produit IV).

On ajoute 2 cm^{**3**} d'éthylèneglycol et 100 mg d'acide paratoluènesulfonique à un mélange de 503 mg de produit obtenu au stade 1 en solution dans 30 cm^{**3**} de dichloroéthane. On porte au reflux pendant 8 heures.

On ajoute 1 cm^{**3**} de triéthylamine et concentre le mélange. On chromatographie sur silice (éluant : acétate d'éthyle-hexane (3-7)). On obtient 450 mg de produit attendu (RF = 0,47) (acétate d'éthyle-hexane (1-1)).

### EXEMPLE 1 : 6alpha-hydroxy 10béta-[2-(méthylthio) éthyl] estra-4,9(11)-diène-3,17-dione

### STADE A : (5béta,6béta,10béta) 3,3,17,17-bis(1,2-éthanediyl) acétal cyclique de 5(6)-époxy 10-(2-hydroxyméthyl) estr-9(11)-ène-3,17-dione (produit A) et (5béta,6béta,9alpha,10béta,11alpha) 3,3,17,17-bis(1,2-éthane-diyl) acétal cyclique de 5(6)-9(11)-diépoxy 10-(2-hydroxy éthyl) estrane-3,17-dione (Produit B).

A un mélange de 4 g du produit obtenu comme indiqué au stade 2 de la préparation 1 avec 800 mg d'acétate de sodium dans 40 cm^{**3**} de chlorure de méthylène, on ajoute à 0°C, 2,08 g d'acide métachloroperbenzoïque à 80 %, agite 30 minutes à 0°C et ajoute à nouveau 2,08 g d'acide métachloroperbenzoïque ; on agite 30 minutes puis hydrolyse le milieu réactionnel par ajout d'une solution aqueuse saturée de bicarbonate de sodium. On extrait avec du chlorure de méthylène, sèche avec du sulfate de magnésium et chromatographie la solution sur silice (éluant : cyclohexane-acétate d'éthyle (7-3) et 1 % de triéthylamine) on obtient 1,85 g de produit A et 1,7 g de produit B.
Spectre de RMN : Produit A (CDCl_{**3**}) 250 MHz.
0,82 ppm (s) 18 méthyl ; 3,17 ppm (d,J ≃ 3) 1H époxyde ; 3,5 à 4,0 ppm les CH_{**2**}O ; 5,50 ppm (m) H_{**11**}.
Spectre de RMN : Produit B (CDCl_{**3**}) 250 MHz.
0,84 ppm (s) 18 méthyl ; 3,05 ppm (d,J = 5) H_{**11**} ; 3,16 ppm (d,J = 2) H_{**6**} alpha ; 3,75 à 4,00 ppm les cétals et CH_{**2**}O.

### STADE B : (5béta,6béta,10béta) 3,3,17,17-bis(1,2-éthanediyl) acétal cyclique de 5(6)-époxy 10-[[2-(méthylsulfonyl) oxy] éthyl] estr-9(11)-ène-3,17-dione.

A une solution de 1,85 g du produit A obtenu au stade A, ci-dessus, dans 20 cm^{**3**} de chlorure de méthylène, on ajoute 0,68 cm^{**3**} de triéthylamine et 0,38 cm^{**3**} de chlorure de méthane sulfonyle. On agite 30 minutes à 0°C puis 1 heure à température ambiante. On ajoute 0,31 cm^{**3**} de triéthylamine et 0,17 cm^{**3**} de chlorure de méthanesulfonyle, on agite 20 minutes et hydrolyse le milieu réactionnel en ajoutant une solution aqueuse saturée de bicarbonate de sodium. On extrait avec du chlorure de méthylène et après évaporation du solvant, on obtient 2,05 g du produit recherché.
Spectre de RMN : (CDCl_{**3**} 300 MHz) ppm.
0,83 (s) 18 CH_{**3**} ; 2,97 (m) SO_{**2**}CH_{**3**} ; 3,8 à 4,0 (m) les cétals ; 4,1 à 4,6 (m) CH_{**2**}OSO_{**2**} ; 5,60-5,55 (m) éthyléniques ; 3,06 (d, J = 3 Hz) 1H époxyde.

### STADE C : (5béta,6béta,10béta) 3,3,17,17-bis(1,2-éthanediyl) acétal cyclique de 5(6)-époxy [2-(méthylthio) éthyl] estr-9(11)-ène-3,17-dione.

A une solution de 1,9 g du produit obtenu au stade B ci-dessus dans 25 cm^{**3**} de diméthyl formamide, on ajoute 1,6 g de thiométhoxyde de sodium et on agite pendant 1 heure à température ambiante. On évapore le diméthyl formamide, reprend le résidu avec du chlorure de méthylène, lave, sèche et évapore à sec. On chromatographie le résidu sur silice (éluant : cyclohexane-acétate d'éthyle (8-2)) on obtient 1,4 g de produit recherché.
Spectre de RMN : (CDCl_{**3**} 300 MHz) ppm.
0,82 (s) 18 CH_{**3**} ; 2,08 (s) S-CH_{**3**} ; 3,0 (d, J ≃ 3) H époxyde ; 3,8 à 4,0 (m) les cétals ; 5,52 (m) H éthyléniques.

### STADE D : 6alpha-hydroxy 10béta-[2-(méthylthio) éthyl] estra-4,9(11)-diène-3,17-dione

On agite 4 heures à température ambiante un mélange de 1 g du produit obtenu au stade C ci-dessus, avec 1 cm^{**3**} de chlorure de méthylène, 10 cm^{**3**} d'éthanol et 5 cm^{**3**} d'une solution d'acide chlorhydrique 1N. On ajoute ensuite une solution aqueuse saturée de bicarbonate de sodium puis on extrait avec du chlorure de méthylène, la solution organique est séchée et concentrée à sec. On chromatographie le résidu sur silice (éluant cyclohexane-acétate d'éthyle (8-2)) on obtient 40,1 mg du produit recherché et 601 mg de 3-(1,2-éthanediyl acétal cyclique) de 5alpha, 6alpha-époxy 10béta-[2-(méthylthio) éthyl] estr-9(11)-èn-3,17-dione.
Spectre de RMN du produit recherché : (CDCl_{**3**} 300 MHz)
0,91 (s) 18 CH_{**3**} ; 2,09 (s) S-CH_{**3**} ; 4,47 (t) après échange H_{**6**} équatorial ; 5,54 (m) H_{**11**} ; 5,90 (s) H_{**4**} ; 0,86 (s) les autres CH_{**3**}.

### EXEMPLE 2 : 9alpha,11alpha-époxy 6béta-hydroxy 10béta-[2-(méthylthio) éthyl] estr-4-ène-3,17-dione

### STADE A : (5béta,6béta,9alpha,10béta,11alpha) 3,3,17,17-bis (1,2-éthanediyl) acétal cyclique de 5(6),9(11)-diépoxy 10-[[2-(méthylsulfonyl) oxy] éthyl] estrane-3,17-dione

A une solution refroidie à 0°C de 1,5 g de produit B obtenu au stade A de l'exemple 1 dans 20 cm^{**3**} de chlorure de méthylène, on ajoute 0,42 cm^{**3**} de chlorure de méthane sulfonyle et 0,75 cm^{**3**} de triéthylamine. On agite 1 heure à 0°C et hydrolyse par ajout d'une solution aqueuse saturée de bicarbonate de sodium. On extrait avec du chlorure de méthylène sèche et évapore à sec. On obtient 1,31 g de produit recherché, utilisé tel quel pour le stade suivant.

### STADE B : (5béta,6béta,9alpha,10béta,11alpha) 3,3,17,17-bis (1,2-éthanediyl) acétal cyclique de 5(6),9(11)-diépoxy 10-[2-(méthylthio) éthyl] estrane-3,17-dione

A une solution de 1 g du produit obtenu au stade A ci-dessus, dans 10 cm^{**3**} de diméthylformamide, on ajoute 931 mg de thiométhoxyde de sodium et agite 2 heures. On évapore le diméthyl formamide reprend avec du chlorure de méthylène, lave, sèche et évapore à sec. On chromatographie le résidu sur silice (éluant : cyclohexane-acétate d'éthyle (8-2)) on obtient 910 mg du produit recherché.
Spectre de RMN : (CDCl_{**3**} 300 MHz) ppm
0,84 (s) 18 CH_{**3**} ; 2,14 (s) S-CH_{**3**} ; 3,08 (d,J = 5,5) H_{**11**} béta ; 3,13 (d,J = 2) H_{**6**} alpha ; 3,75 à 4,00 les cétals.

### STADE C : 9alpha,11alpha-époxy 6béta-hydroxy 10béta-[2-(méthylthio) éthyl] estr-4-ène-3,17-dione

On agite 72 heures à température ambiante 700 mg du produit obtenu au stade B ci-dessus, avec 15 cm^{**3**} d'éthanol et 2 cm^{**3**} d'acide chlorhydrique 2N. On hydrolyse par ajout d'une solution aqueuse de bicarbonate de sodium, extrait avec du chlorure de méthylène, sèche et évapore à sec. On chromatographie le résidu sur silice (éluant : cyclohexane-acétate d'éthyle (8-2)), on obtient 335 mg du produit recherché et 98,6 mg de 3-(1,2-éthanediyl acétal cyclique) de 5béta, 6béta, 9alpha, 11alpha-diépoxy 10béta-[2-(méthylthio) éthyl] estran-3,17-dione.
Spectre de RMN du produit recherché : (CDCl_{**3**} 300 MHz) ppm 0,99 (s) 18 CH_{**3**} ; 2,15 (s) S-CH_{**3**} ; 3,25 (t,l) H_{**11**} éq. ; 4,52 (t,l) H_{**6**} éq. ; 5,96 (t,l) H_{**4**}.

### Etude pharmacologique des produits de l'invention.

Inhibition dépendante de la concentration (mesure de la CI_{**50**} = concentration de l'inhibiteur nécessaire pour réduire l'activité enzymatique de 50 %).

On utilise des placentas humains qui, une heure au plus après l'accouchement sont lavés, perfusés par du sérum physiologique (5 litres) via la veine ombilicale puis congelés à -40°C.

### 1) Obtention des microsomes placentaires

Les placentas sont décongelés à 4°C puis homogénéisés (1:3) dans un tampon phosphate 10 mM, pH = 7,0 ; contenant 100 millimoles de chlorure de potassium (KCl), 10 millimoles de dithiothreitol (DTT), 10 millimoles d'acide éthylènediaminetétraacétique (EDTA), 40 millimoles de nicotinamide et 250 millimoles de sucrose.

Les homogénats sont ensuite soumis à différentes phases de centrifugation jusqu'à l'obtention du surnageant "9000 g" (correspondant au cytosol et au réticulum endoplasmique).

Ce surnageant est alors soumis à une étape d'ultracentrifugation (1 heure 30 minutes, 105000 g) pour obtenir le culot microsomal.

Les microsomes sont alors resuspendus dans un tampon phosphate 50 millimoles, pH = 7,4, contenant 100 millimoles KCl, 1 millimole EDTA, 1 millimole DTT et du glycérol (10 %).

La suspension microsomale est ensuite aliquotée et les fractions congelées à la température de l'azote liquide.

La concentration en protéines de la suspension microsomale est déterminée par la méthode de BRADFORD (BRADFORD M., Anal. Biochem., 72, (1976) 248).

### 2) Mesure de la CI₅₀de chaque inhibiteur

A 960 microlitres de tampon phosphate (50 millimoles, pH = 7,2), 2,5 millimoles glucose-6-phosphate, et contenant 0,16 unité internationale de glucose-6-phosphate déshydrogénase (G-6-PDH), on ajoute dans l'ordre :
1° - 10 microlitres d'inhibiteur, solubilisé dans le diméthylsulfoxyde (DMSO), pour donner des concentrations finales allant de 10^{**-5**}M à 10^{**-10**}M.
2° - 10 microlitres de substrat. Le substrat est de l'Androstènedione 500 nM solubilisée dans l'éthanol et contenant de la 1béta-2béta-(^{**3**}H)-Androstènedione à dilution isotopique connue (≃ 200.000 désintégrations par minute).
3° - 10 microlitres de suspension microsomale, équivalant à 25 microgrammes de protéines par test.

La réaction enzymatique est alors très rapidement initiée par un ajout de 10 microlitres de nicotinamide adénine dinucléotide phosphate réduit (NADPH) solubilisé dans l'eau.

Après agitation, chaque test est incubé à 37°C pendant 10 minutes. La réaction est ensuite stoppée par un ajout de chloroforme (4 ml).

Après agitation vigoureuse des tubes, ceux-ci sont décantés et centrifugés à 4°C pendant 10 minutes à la vitesse de 3000 r.p.m. (rotations par minutes) soit 600 X g.

Après centrifugation, et pour chaque tube, 100 micro-litres de surnageant sont prélevés et mis à compter en présence de liquide scintillant.

Cette méthode est dérivée des procédures décrites par REED et Coll. (J.Biol. Chem., 251, (1976), 1625) et THOMPSON et Coll. (J.Biol. Chem., 249, (1974), 5364).

L'activité enzymatique (aromatase), est proportionnelle au pourcentage de tritium relargué sous forme d'eau tritiée (^{**3**}H_{**2**}O) au cours de la réaction.

L'inhibition obtenue pour chaque concentration de chaque produit inhibiteur de l'invention est calculée comme un pourcentage des contrôles (100 % arbitraire, obtenus en absence de tout inhibiteur).

La CI_{**50**} est égale à la concentration d'inhibiteur nécessaire pour diminuer de 50 % l'activité enzymatique.

Les valeurs des CI_{**50**} obtenues pour les produits inhibiteurs de l'invention sont les suivantes :
Produit de l'exemple 2 : CI_{**50**} = 1,8.10⁻^{**7**}M.

## Revendications (Revendications pour l'(les) Etat(s) contractant(s) suivant(s): AT, BE, CH, DE, DK, FR, GB, IT, LI, LU, NL, PT, SE)

1. Les produits de formule générale (I) : 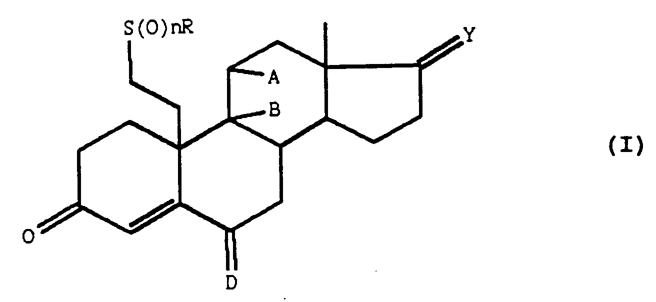 dans laquelle le substituant R représente un radical alkyle, alcényle ou alcynyle ayant au plus 4 atomes de carbone,
Y représente un atome d'oxygène ou un reste : 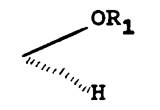 dans lequel R_{**1**} représente un atome d'hydrogène ou un radical acyle,
n représente un entier de 0 à 2,
D représente un atome d'oxygène ou un reste 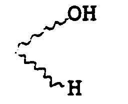 A et B forment ensemble une fonction époxyde alpha ou avec les atomes de carbone auxquels ils sont liés, une seconde liaison entre ces carbones.

2. Les produits de formule générale (I′) : 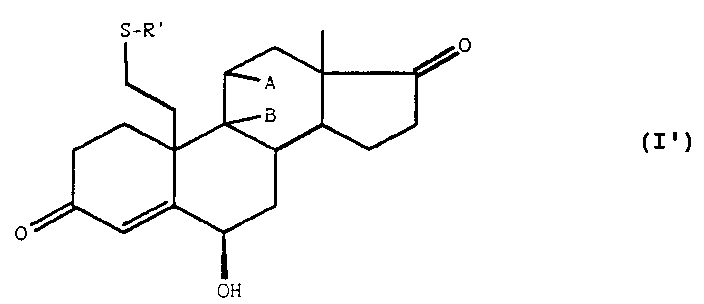 dans laquelle A et B ont la signification précédente et R′ représente un radical alkyle ayant au plus 4 atomes de carbone.

3. Les produits de formule générale (I′) telle que définie à la revendication 2, répondant aux formules :
- 6béta-hydroxy 10béta-[2-(méthylthio) éthyl] estra-4,9(11)-diène-3,17-dione,
- 9alpha,11alpha-époxy 6béta-hydroxy 10béta-[2-(méthylthio) éthyl] estr-4-ène-3,17-dione.

4. Procédé de préparation des produits de formule générale (I) telle que définie à la revendication 1, caractérisé en ce que l'on soumet un produit de formule (II) : 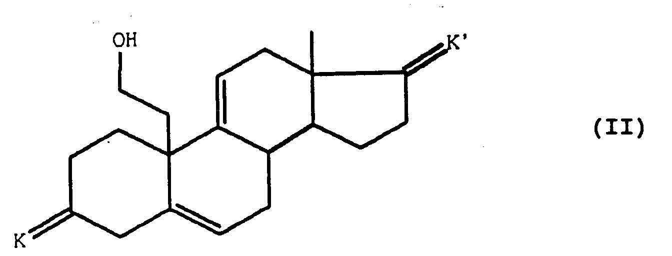 dans laquelle K et K′, identiques ou différents représentent un radical céto protégé, à l'action d'un réactif d'époxydation pour obtenir les produits de formule (IIIa) et (IIIb) : 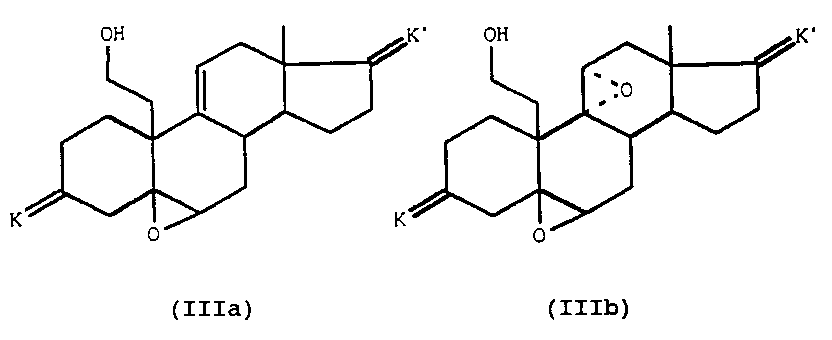 produits que l'on traite par un réactif de transformation du radical hydroxy en radical thiol pour obtenir des produits de formules (IVa) et (IVb) : 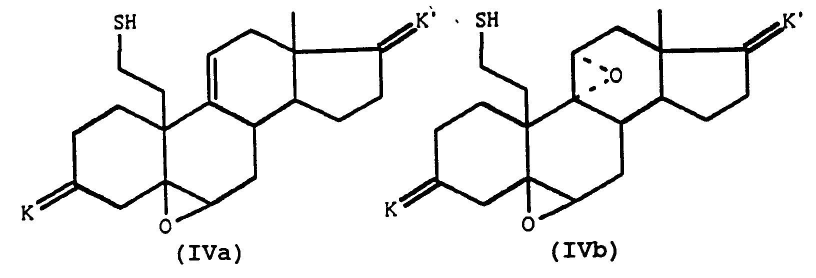 que l'on traite par un dérivé réactif du radical R, R ayant la définition donnée à la revendication 1 pour obtenir les produits de formules (Va) et (Vb) : 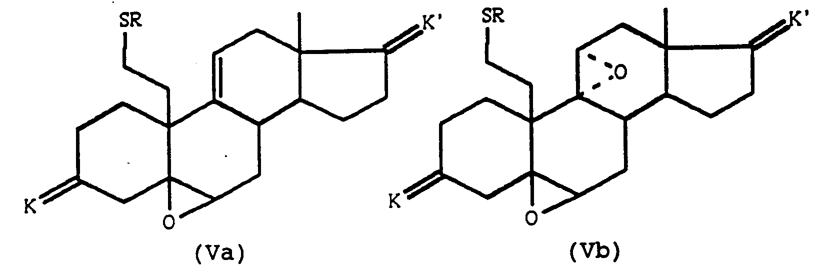 produits de formules (Va) et (Vb) que liaison traite par un réactif de déprotection des fonctions cétones protégées et d'ouverture de l'époxyde en position 5(6) pour obtenir des produits de formules (I′a) et (I′b) : 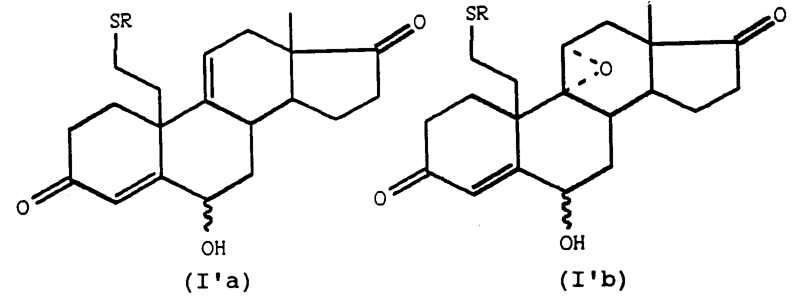 correspondant aux produits de formule (I) dans laquelle R a les significations indiquées à la revendication 1, Y représente un atome d'oxygène, n représente le nombre 0, et D représente un reste : 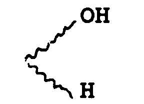 produits de formule (I′a) et (I′b) que, si désiré, l'on soumet à l'une quelconque des réactions suivantes :
a) réduction suivie éventuellement d'une acylation de la fonction cétone en position 17,
b) oxydation de l'atome de soufre du radical -SR en sulfoxyde ou en sulfone,
c) oxydation de la fonction hydroxyle en position 6 en cétone.

5. Procédé de préparation des produits de formule générale (I) telle que définie à la revendication 1, caractérisé en ce que l'on soumet un produit de formule (II) : 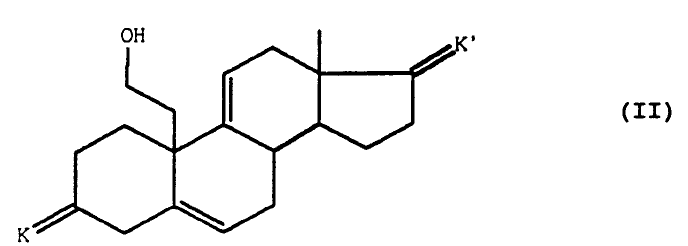 dans laquelle K et K′, identiques ou différents représentent un radical céto protégé, à l'action d'un réactif d'époxydation pour obtenir les produits de formule (IIIa) et (IIIb) : 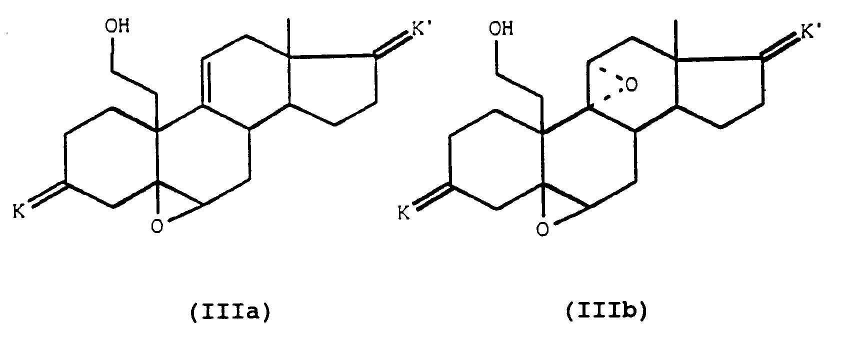 produits que l'on traite par un dérivé réactif d'un acide sulfonique pour obtenir les produits de formules (VIa) et (VIb) : 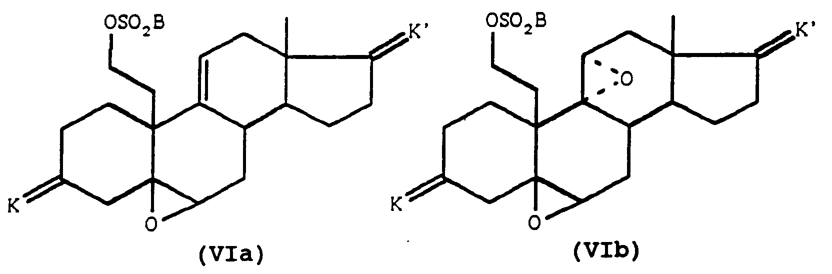 dans laquelle B représente le reste d'un acide sulfonique produits de formules (VIa) et (VIb) que l'on traite par un sel de formule RSA dans laquelle A représente un cation monovalent et R ayant la définition donnée à la revendication 1, pour obtenir des produits de formules (Va) et (Vb) : 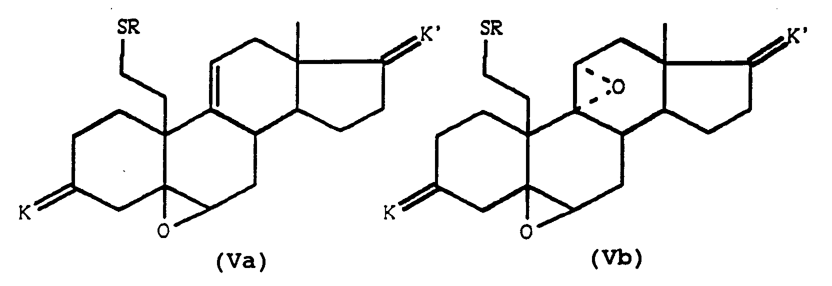 produits de formules (Va) et (Vb) que l'on traite par un réactif de déprotection des fonctions cétones protégées et d'ouverture de l'époxyde en position 5(6) pour obtenir des produits de formules (I′a) et (I′b) : 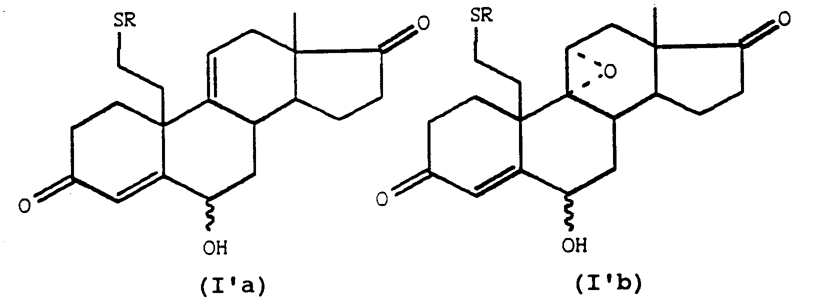 correspondant aux produits de formule (I) dans laquelle R a les significations indiquées à la revendication 1, Y représente un atome d'oxygène, n représente le nombre 0, et D représente un reste : 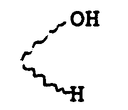 produits de formule (I′a) et (I′b) que, si désiré, l'on soumet à l'une quelconque des réactions suivantes :
a) réduction suivie éventuellement d'une acylation de la fonction cétone en position 17,
b) oxydation de l'atome de soufre du radical -SR en sulfoxyde ou en sulfone,
c) oxydation de la fonction hydroxyle en position 6 en cétone.

6. Procédé selon l'une des revendications 4 et 5, caractérisé en ce que la transformation des produits de formules (Va) et (Vb) est effectuée dans des conditions ménagées pour obtenir les produits de formules (VIIa) et (VIIb) : 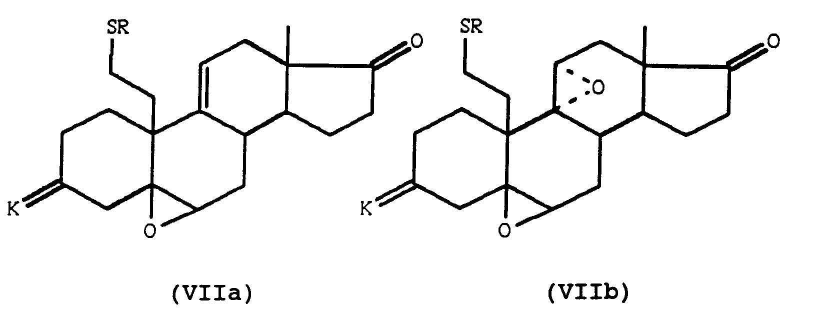 produits de formules (VIIa) et (VIIb) que l'on traite par un réactif de déprotection de la fonction cétone et d'ouverture de l'époxyde en position 5(6) pour obtenir les produits de formules (I′a) et (I′b) telles que définies à la revendication 5.

7. A titre de médicaments les produits de formule générale (I) telle que décrite à la revendication 1.

8. A titre de médicaments les produits de formule générale (I′) telle que définie à l'une des revendication 2 ou 3.

9. Les compositions pharmaceutiques contenant, à titre de principe actif l'un au moins des médicaments définis à l'une des revendications 7 ou 8.

10. A titre de produits industriels nouveaux, les produits de formules générales (IIIa), (IIIb), (IVa), (IVb), (Va), (Vb), tels que définis à la revendication 4 les produits de formules générales ((VIa), (VIb) tels que définis à la revendication 5 et les produits de formules générales (VIIa) et (VIIb) tels que définis à la revendication 6.

## Revendications (Revendications pour l'(les) Etat(s) contractant(s) suivant(s): ES)

1. Procédé pour préparer les produits de formule générale (I) : 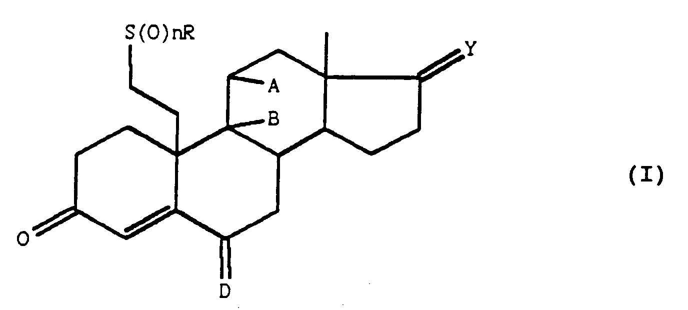 dans laquelle le substituant R représente un radical alkyle, alcényle ou alcynyle ayant au plus 4 atomes de carbone,
Y représente un atome d'oxygène ou un reste : 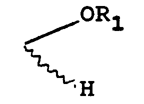 dans lequel R_{**1**} représente un atome d'hydrogène ou un radical acyle,
n représente un entier de 0 à 2,
D représente un atome d'oxygène ou un reste 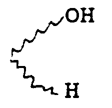 A et B forment ensemble une fonction époxyde alpha ou avec les atomes de carbone auxquels ils sont liés, une seconde liaison entre ces carbones, caractérisé en ce que l'on soumet un produit de formule (II) : 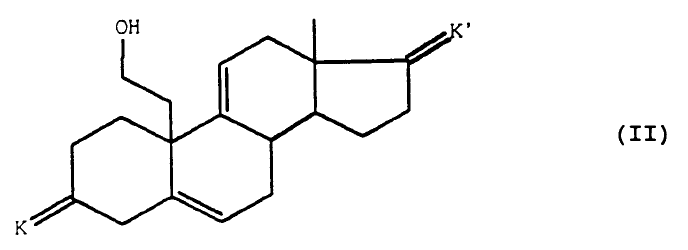 dans laquelle K et K′, identiques ou différents représentent un radical céto protégé, à l'action d'un réactif d'époxydation pour obtenir les produits de formule (IIIa) et (IIIb) : 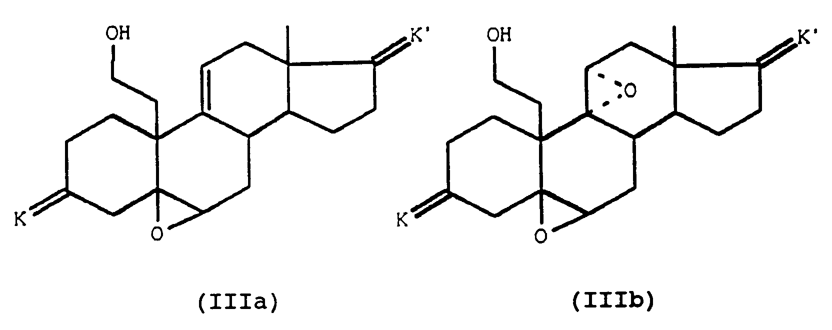 produits que l'on traite par un réactif de transformation du radical hydroxy en radical thiol pour obtenir des produits de formules (IVa) et (IVb) : 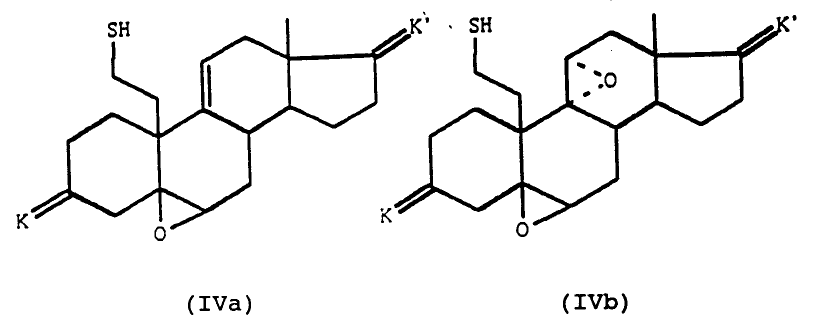 que l'on traite par un dérivé réactif du radical R, R ayant la définition indiquée ci-dessus pour obtenir les produits de formules (Va) et (Vb) : 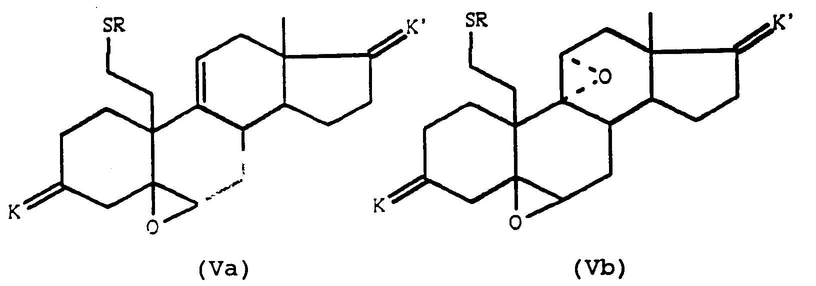 ou bien par un dérivé réactif d'un acide sulfonique pour obtenir les produits de formules (VIa) et (VIb) : 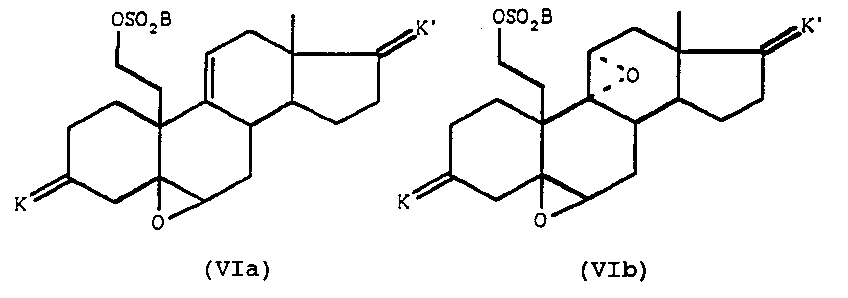 dans laquelle B représente le reste d'un acide sulfonique produits de formules (VIa) et (VIb) que l'on traite par un sel de formule RSA dans laquelle A représente un cation monovalent pour obtenir des produits de formules (Va) et (Vb) telles que définies ci-dessus, produits de formules (Va) et (Vb) que l'on traite par un réactif de déprotection des fonctions cétones protégées et d'ouverture de l'époxyde en position 5(6) pour obtenir des produits de formules (I′a) et (I′b) : 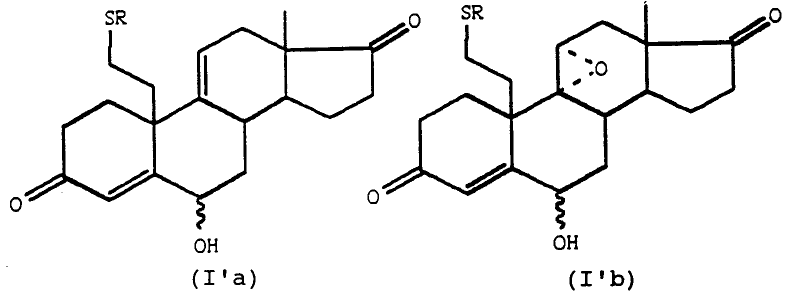 correspondant aux produits de formule (I) dans laquelle R a les significations indiquées ci-dessus, Y représente un atome d'oxygène, n représente le nombre 0, et D représente un reste : 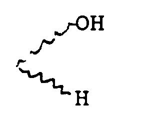 produits de formule (I′a) et (I′b) que, si désiré, l'on soumet à l'une quelconque des réactions suivantes :
a) réduction suivie éventuellement d'une acylation de la fonction cétone en position 17,
b) oxydation de l'atome de soufre du radical -SR en sulfoxyde ou en sulfone,
c) oxydation de la fonction hydroxyle en position 6 en cétone.

2. Procédé selon la revendication 1, caractérisé en ce que la transformation des produits de formules (Va) et (Vb) est effectuée dans des conditions ménagées pour obtenir les produits de formules (VIIa) et (VIIb) : 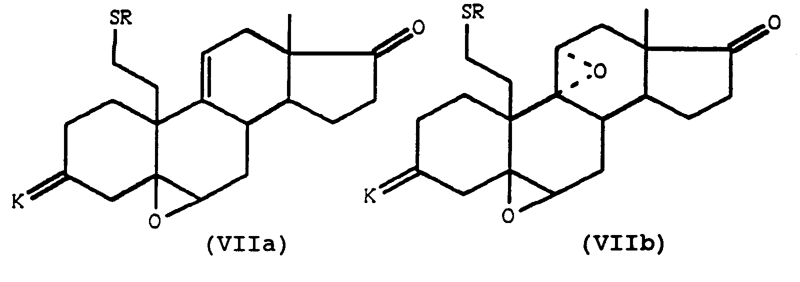 produits de formules (VIIa) et (VIIb) que l'on traite par un réactif de déprotection de la fonction cétone et d'ouverture de l'époxyde en position 5(6) pour obtenir les produits de formules (I′a) et (I′b) telles que définies à la revendication 1.

3. Procédé selon la revendication 1 ou 2 pour préparer des produits de formule (I) répondant à la formule générale (I′) : 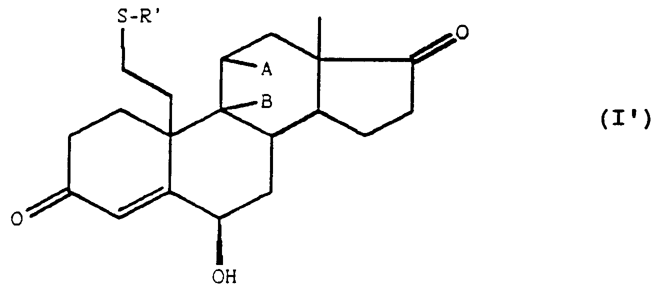 dans laquelle A et B ont la signification précédente et R′ représente un radical alkyle ayant au plus 4 atomes de carbone caractérisé en ce que l'on utilise comme dérivé réactif que l'on fait agir sur le produit de formule (IV_{**a**}) ou (IV_{**b**}) ou comme sel de formule RSA que l'on fait agir sur le produit de formule (VI_{**a**}) ou (VI_{**b**}) un produit dans lequel R représente un radical alkyle ayant au plus 4 atomes de carbone.

## Revendications (Revendications pour l'(les) Etat(s) contractant(s) suivant(s): GR)

1. Procédé pour préparer les produits de formule générale (I) : 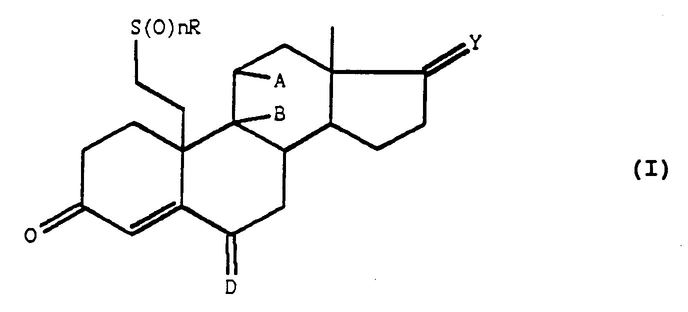 dans laquelle le substituant R représente un radical alkyle, alcényle ou alcynyle ayant au plus 4 atomes de carbone,
Y représente un atome d'oxygène ou un reste : 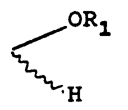 dans lequel R_{**1**} représente un atome d'hydrogène ou un radical acyle,
n représente un entier de 0 à 2,
D représente un atome d'oxygène ou un reste 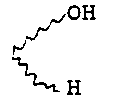 A et B forment ensemble une fonction époxyde alpha ou avec les atomes de carbone auxquels ils sont liés, une seconde liaison entre ces carbones, caractérisé en ce que l'on soumet un produit de formule (II) : 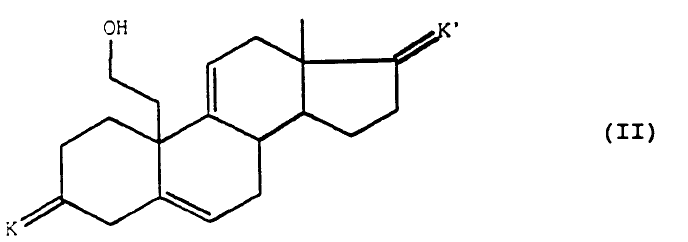 dans laquelle K et K′, identiques ou différents représentent un radical céto protégé, à l'action d'un réactif d'époxydation pour obtenir les produits de formule (IIIa) et (IIIb) : 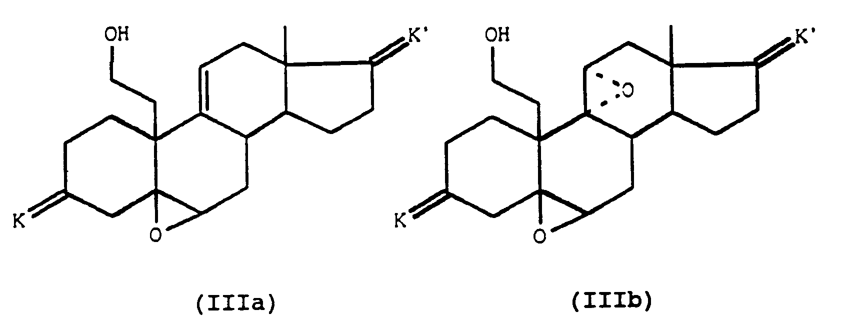 produits que l'on traite par un réactif de transformation du radical hydroxy en radical thiol pour obtenir des produits de formules (IVa) et (IVb) : 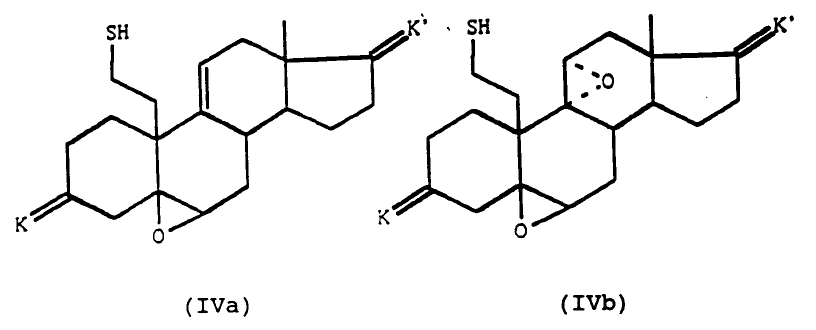 que l'on traite par un dérivé réactif du radical R, R ayant la définition indiquée ci-dessus pou obtenir les produits de formules (Va) et (Vb) : 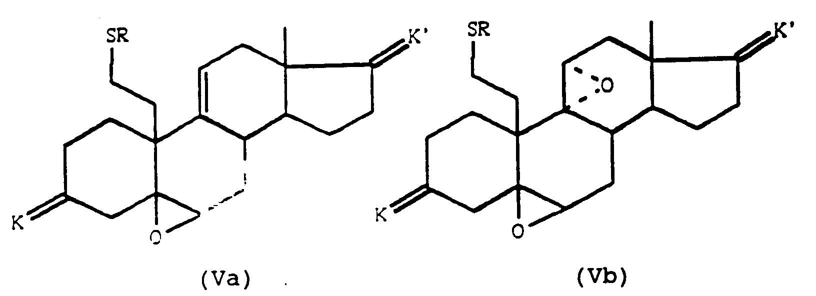 ou bien par un dérivé réactif d'un acide sulfonique pour obtenir les produits de formules (VIa) et (VIb) : 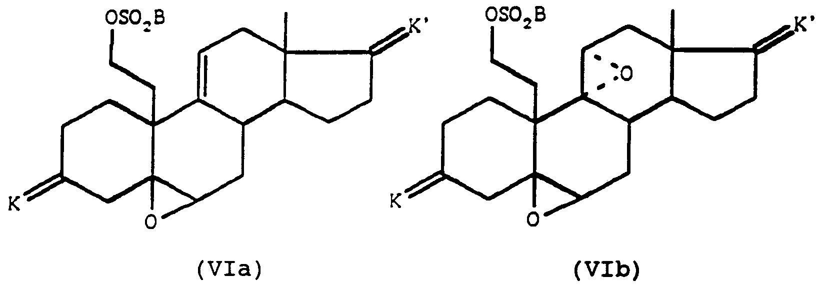 dans laquelle B représente le reste d'un acide sulfonique produits de formules (VIa) et (VIb) que l'on traite par un sel de formule RSA dans laquelle A représente un cation monovalent pour obtenir des produits de formules (Va) et (Vb) telles que définies ci-dessus, produits de formules (Va) et (Vb) que l'on traite par un réactif de déprotection des fonctions cétones protégées et d'ouverture de l'époxyde en position 5(6) pour obtenir des produits de formules (I′a) et (I′b) : 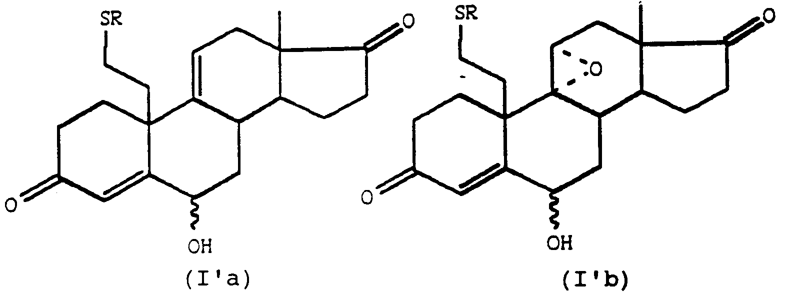 correspondant aux produits de formule (I) dans laquelle R a les significations indiquées ci-dessus, Y représente un atome d'oxygène, n représente le nombre 0, et D représente un reste : 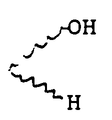 produits de formule (I′a) et (I′b) que, si désiré, l'on soumet à l'une quelconque des réactions suivantes :
a) réduction suivie éventuellement d'une acylation de la fonction cétone en position 17,
b) oxydation de l'atome de soufre du radical -SR en sulfoxyde ou en sulfone,
c) oxydation de la fonction hydroxyle en position 6 en cétone.

2. Procédé selon la revendication 1, caractérisé en ce que la transformation des produits de formules (Va) et (Vb) est effectuée dans des conditions ménagées pour obtenir les produits de formules (VIIa) et (VIIb) : 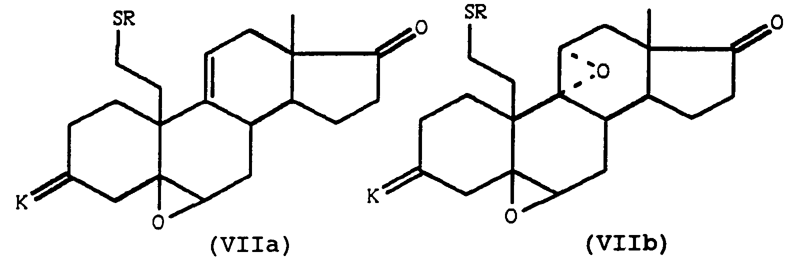 produits de formules (VIIa) et (VIIb) que l'on traite par un réactif de déprotection de la fonction cétone et d'ouverture de l'époxyde en position 5(6) pour obtenir les produits de formules (I′a) et (I′b) telles que définies à la revendication 1.

3. Procédé selon la revendication 1 ou 2 pour préparer des produits de formule (I) répondant à la formule générale (I′) : 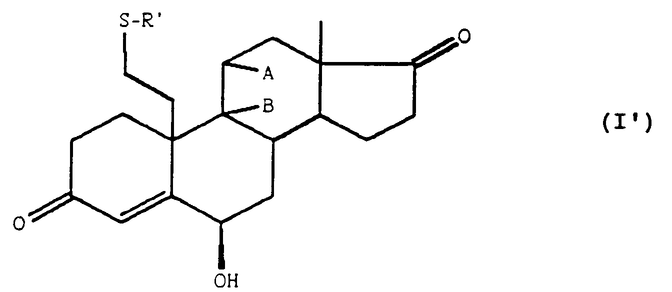 dans laquelle A et B ont la signification précédente et R′ représente un radical alkyle ayant au plus 4 atomes de carbone caractérisé en ce que l'on utilise comme dérivé réactif que l'on fait agir sur le produit de formule (IV_{**a**}) ou (IV_{**b**}) ou comme sel de formule RSA que l'on fait agir sur le produit de formule (VI_{**a**}) ou (VI_{**b**}) un produit dans lequel R représente un radical alkyle ayant au plus 4 atomes de carbone.

4. Procédé selon l'une quelconque des revendications 1 à 3, caractérisé en ce que l'on choisit les réactifs de manière telle que l'on prépare l'un des produits répondant aux formules :
- 6béta-hydroxy 10béta-[2-(méthylthio) éthyl] estra-4,9(11)-diène-3,17-dione,
- 9alpha,11alpha-époxy 6béta-hydroxy 10béta-[2-(méthylthio) éthyl] estr-4-ène-3,17-dione.

5. Procédé de préparation de compositions pharmaceutiques, caractérisé en ce que liaison met à titre de principe actif, l'un au moins des produits de formule générale (I) telle que définie à la revendication 1 sous une forme destinée à cet usage.

6. Procédé de préparation de compositions pharmaceutiques, caractérisé en ce que l'on met à titre de principe actif, l'un au moins des produits de formule générale (I) telle que définie à l'une des revendications 2 à 4 sous une forme destinée à cet usage.

7. A titre de produits industriels nouveaux, les produits de formules générales (IIIa), (IIIb), (IVa), (IVb), (Va), (Vb), (VI_{**a**}) et (VI_{**b**}) tels que définis à la revendication 1 et les produits de formules générales (VIIa) et (VIIb) tels que définis à la revendication 2.

## Claims (Claims for the following Contracting State(s): AT, BE, CH, DE, DK, FR, GB, IT, LI, LU, NL, PT, SE)

1. The products of general formula (I): 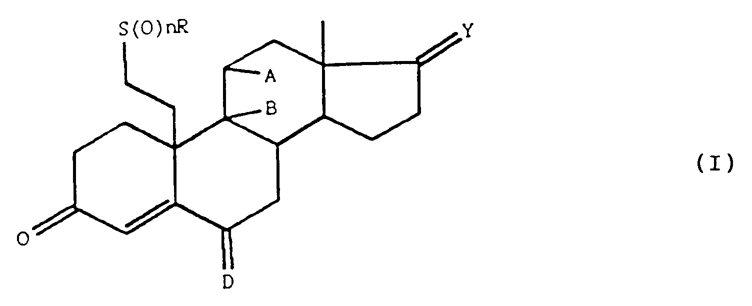 in which the substituent R represents an alkyl, alkenyl or alkynyl radical having at most 4 carbon atoms, Y represents an oxygen atom or a remainder: 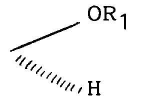 in which R₁ represents a hydrogen atom or an acyl radical,
n represents an integer from 0 to 2,
D represents an oxygen atom or a remainder 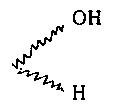 A and B together form an alpha epoxide function or with the carbon atoms to which they are linked, a second bond between these carbons.

2. The products of general formula (I'): 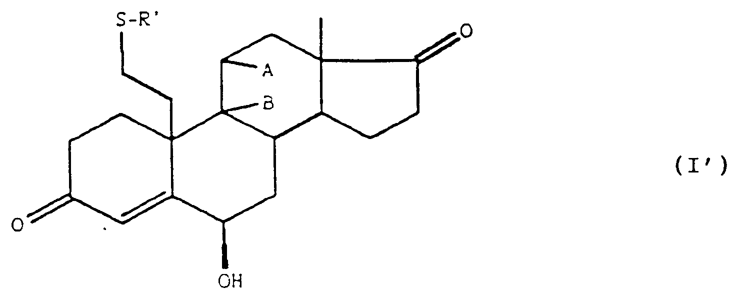 in which A and B have the previous meaning and R' represents an alkyl radical having at most 4 carbon atoms.

3. The products of general formula (I ') as defined in claim 2, corresponding to formulae:
- 6beta-hydroxy-10beta-[2-(methylthio)-ethyl]-estra-4,9(11)-diene-3,17-dione,
- 9alpha,11alpha-epoxy-6beta-hydroxy-10beta-[2-(methylthio)-ethyl]-estr-4-ene-3,17-dione.

4. Preparation process for the products of general formula (I) as defined in claim 1, characterized in that a product of formula (II): 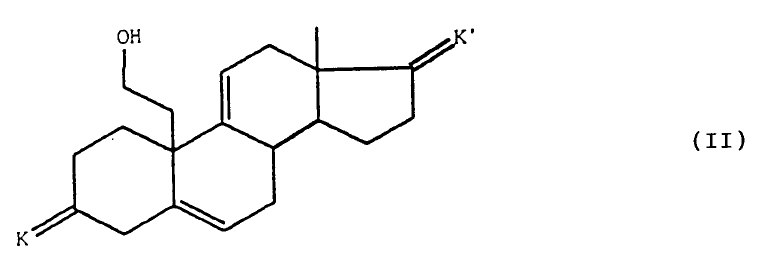 in which K and K', identical or different, represent a protected keto radical, is subjected to the action of an epoxidation reagent in order to obtain the products of formula (IIIa) and (IIIb): 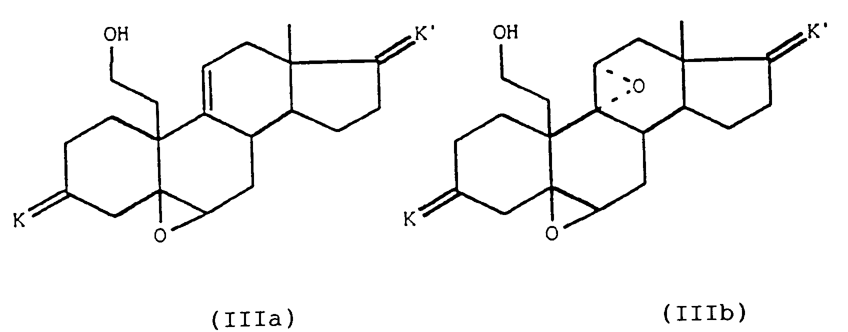 which products are treated with a conversion reagent of the hydroxy radical into a thiol radical in order to obtain the products of formulae (IVa) and (IVb): 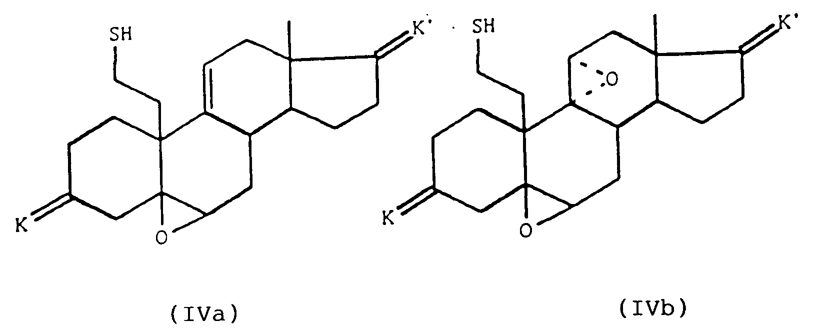 which are treated with a reactive derivative of the R radical, R having the meaning given in claim 1, in order to obtain the products of formulae (Va) and (Vb): 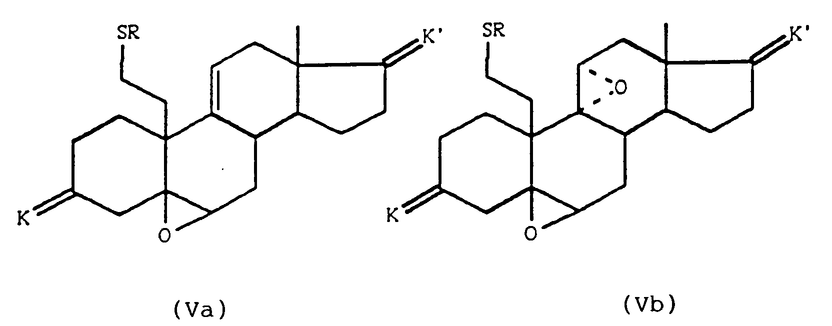 which products of formulae (Va) and (Vb) are treated with a deprotection reagent of the protected ketone functions and opening of the epoxide in position 5(6) in order to obtain the products of formulae (I'a) and (I'b): 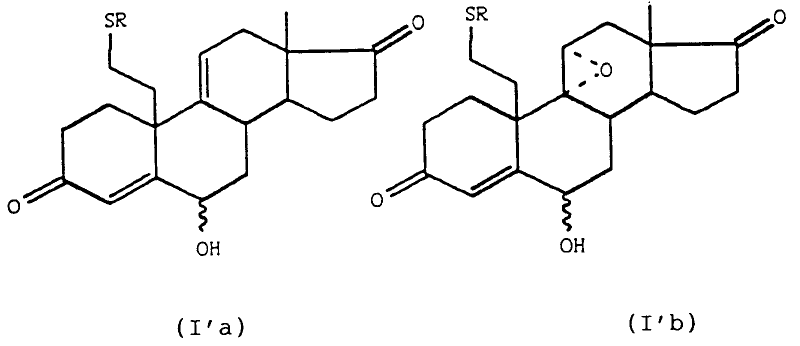 corresponding to products of formula (I) in which R has the meanings indicated in claim 1, Y represents an oxygen atom, n represents the number 0, and D represents a remainder: 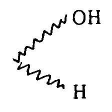 which products of formula (I'a) and (I'b) are, if desired, subjected to any one of the following reactions:
a) reduction optionally followed by an acylation of the ketone function in position 17,
b) oxidation of the sulphur atom of the -SR radical into the sulphoxide or sulphone,
c) oxidation of the hydroxyl function in position 6 into the ketone.

5. Preparation process for the products of general formula (I) as defined in claim 1, characterized in that a product of formula (II): 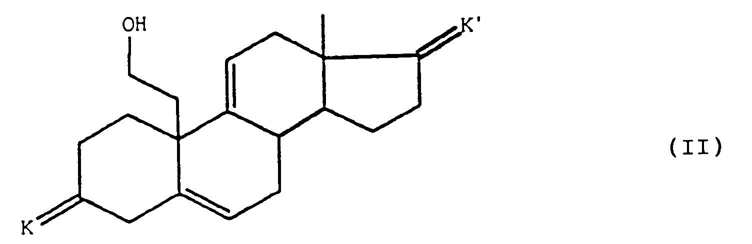 in which K and K', identical or different, represent a protected keto radical, is subjected to the action of an epoxidation reagent in order to obtain the products of formula (IIIa) and (IIIb): 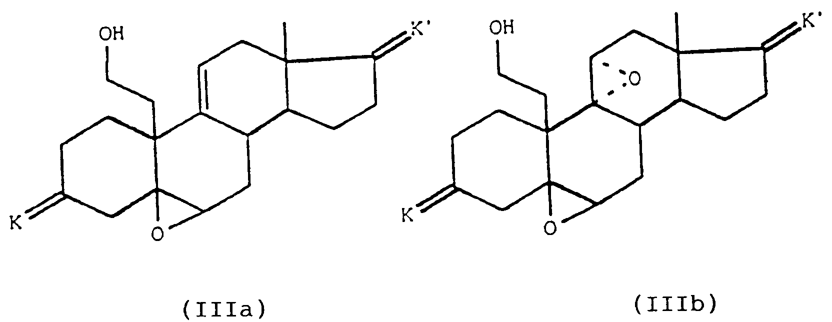 which products are treated by a reactive derivative of a sulphonic acid in order to obtain the products of formulae (VIa) and (VIb): 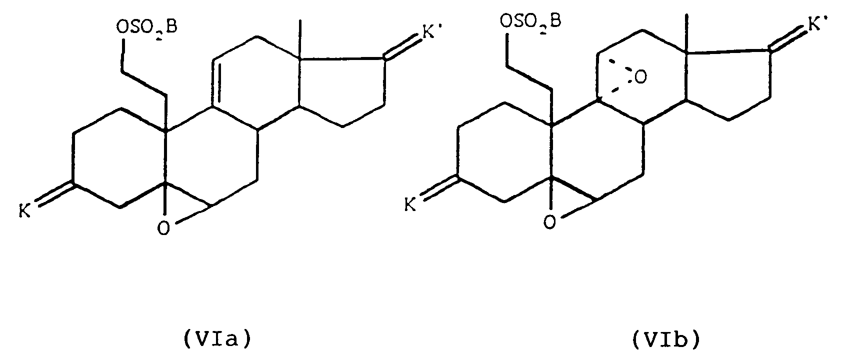 in which B represents the remainder of a sulphonic acid, which products of formulae (VIa) and (VIb) are treated with a salt of formula RSA in which A represents a monovalent cation and R having the definition given in claim 1, in order to obtain products of formulae (Va) and (Vb): 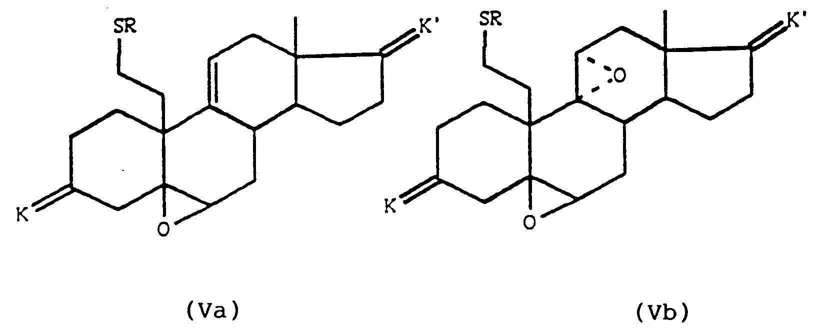 which products of formulae (Va) and (Vb) are treated with a deprotection reagent of the protected ketone functions and opening of the epoxide in position 5(6) in order to obtain the products of formulae (I'a) and (I'b): 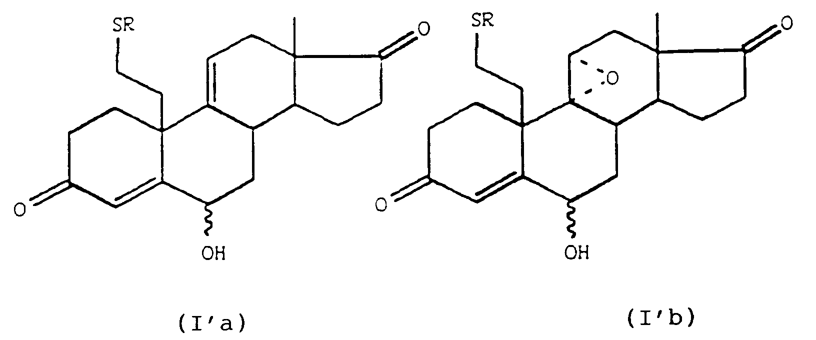 corresponding to products of formula (I) in which R has the meanings indicated in claim 1, Y represents an oxygen atom, n represents the number 0, and D represents a remainder: 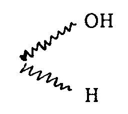 which products of formula (I'a) and (I'b) are, if desired, subjected to any one of the following reactions:
a) reduction optionally followed by an acylation of the ketone function in position 17,
b) oxidation of the sulphur atom of the -SR radical into the sulphoxide or sulphone,
c) oxidation of the hydroxyl function in position 6 into the ketone.

6. Process according to one of claims 4 and 5, characterized in that the conversion of products of formulae (Va) and (Vb) is carried out under carefully-controlled conditions to obtain the products of formulae (VIIa) and (VIIb): 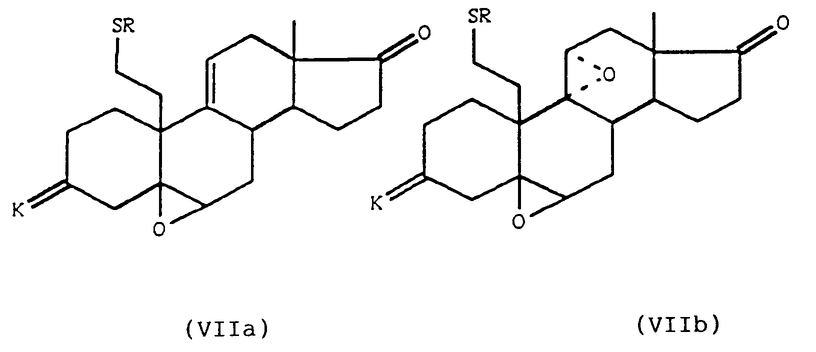 which products of formulae (VIIa) and (VIIb) are treated with a deprotection reagent of the ketone function and opening of the epoxide in position 5(6) in order to obtain the products of formulae (I'a) and (I'b) as defined in claim 5.

7. As medicaments, the products of general formula (I) as described in claim 1.

8. As medicaments, the products of general formula (I') as defined in one of claims 2 or 3.

9. Pharmaceutical composition containing, as active ingredient, at least one of the medicaments defined in one of claims 7 or 8.

10. As new industrial products, the products of general formulae (IIIa), (IIIb), (IVa), (IVb), (Va), (Vb), as defined in claim 4, the products of general formulae ((VIa), (VIb) as defined in claim 5 and the products of general formulae (VIIa) and (VIIb) as defined in claim 6.

## Claims (Claims for the following Contracting State(s): ES)

1. Preparation process for the products of general formula (I): 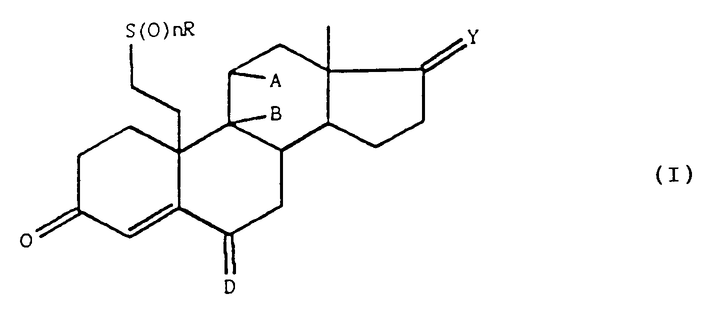 in which the substituent R represents an alkyl, alkenyl or alkynyl radical having at most 4 carbon atoms, Y represents an oxygen atom or a remainder: 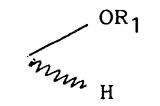 in which R₁ represents a hydrogen atom or an acyl radical,
n represents an integer from 0 to 2,
D represents an oxygen atom or a remainder 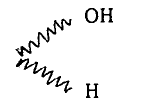 A and B together form an alpha epoxide function or with the carbon atoms to which they are linked, a second bond between these carbons, characterized in that a product of formula (II): 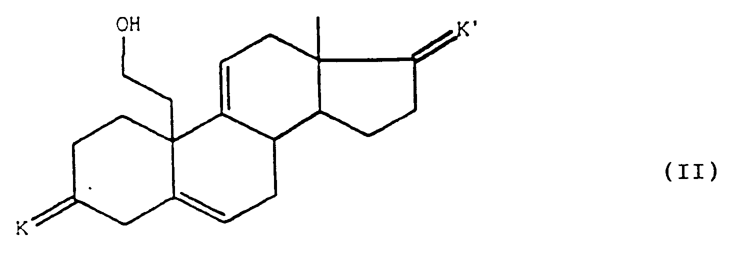 in which K and K', identical or different, represent a protected keto radical, is subjected to the action of an epoxidation reagent in order to obtain the products of formula (IIIa) and (IIIb): 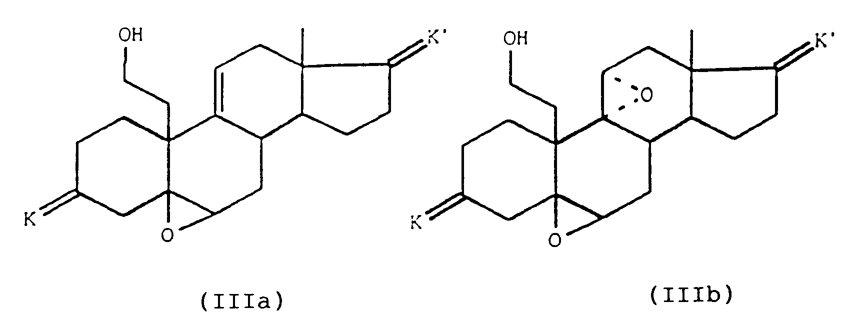 which products are treated with a conversion reagent of the hydroxy radical into a thiol radical in order to obtain the products of formulae (IVa) and (IVb): 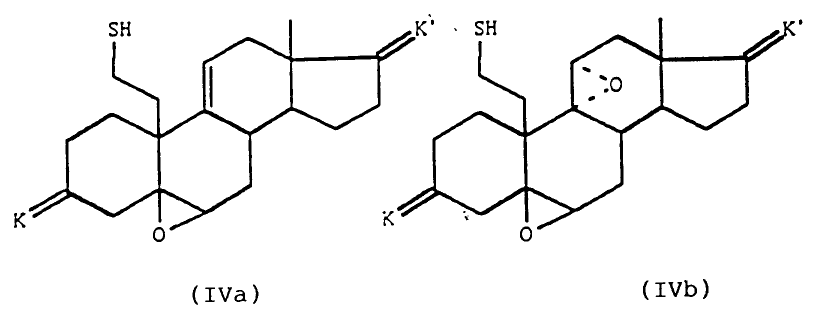 which are treated with a reactive derivative of the R radical, R having the meaning indicated above, in order to obtain the products of formulae (Va) and (Vb): 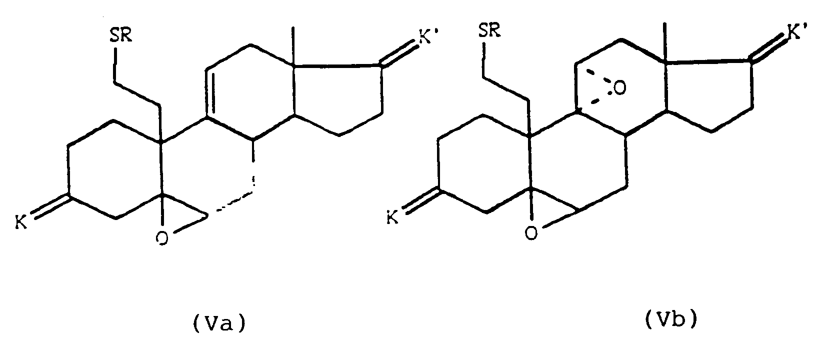 or with a reactive derivative of a sulphonic acid in order to obtain the products of formulae (VIa) and (VIb): 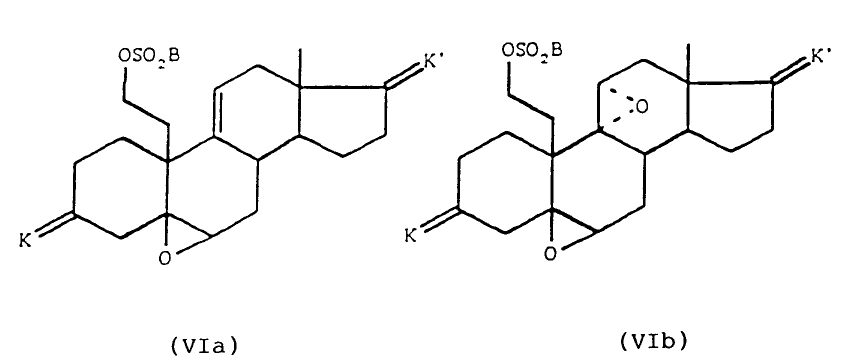 in which B represents the remainder of a sulphonic acid, which products of formulae (VIa) and (VIb) are treated with a salt of formula RSA in which A represents a monovalent cation in order to obtain products of formulae (Va) and (Vb) as defined above which products of formulae (Va) and (Vb) are treated with a deprotection reagent of the protected ketone functions and opening of the epoxide in position 5(6) in order to obtain the products of formulae (I'a) and (I'b): 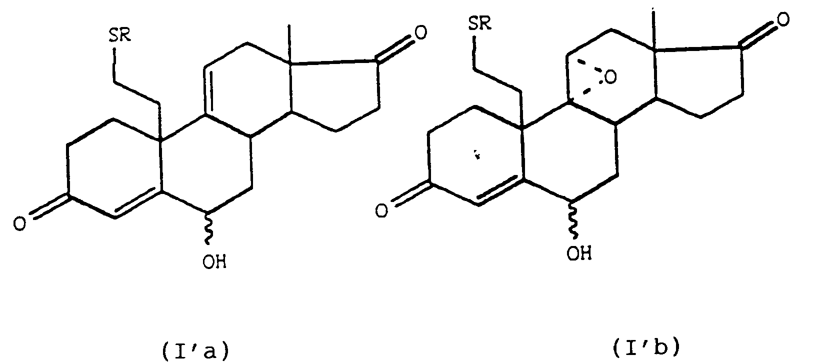 corresponding to products of formula (I) in which R has the meanings indicated in claim 1, Y represents an oxygen atom, n represents the number 0, and D represents a remainder: 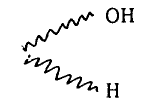 which products of formula (I'a) and (I'b) are, if desired, subjected to any one of the following reactions:
a) reduction optionally followed by an acylation of the ketone function in position 17,
b) oxidation of the sulphur atom of the -SR radical into the sulphoxide or sulphone,
c) oxidation of the hydroxyl function in position 6 into the ketone.

2. Process according to claim 1, characterized in that the conversion of the products of formulae (Va) and (Vb) is carried out under carefully-controlled conditions in order to obtain the products of formulae (VIIa) and (VIIb): 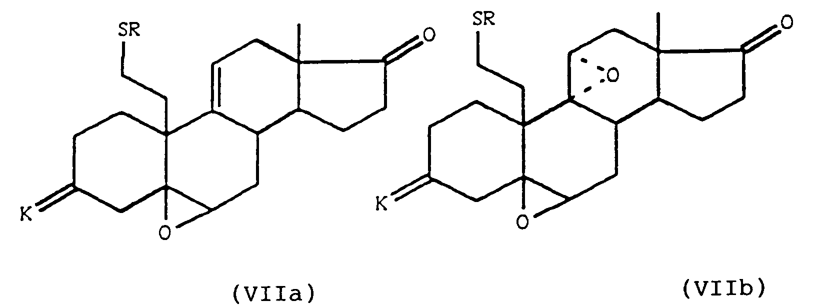 which products of formulae (VIIa) and (VIIb) are treated with a deprotection reagent of the ketone function and opening of the epoxide in position 5(6) in order to obtain the products of formulae (I'a) and (I'b) as defined in claim 1.

3. Process according to claim 1 or 2 for the preparation of products of formula (I) corresponding to general formula (I'): 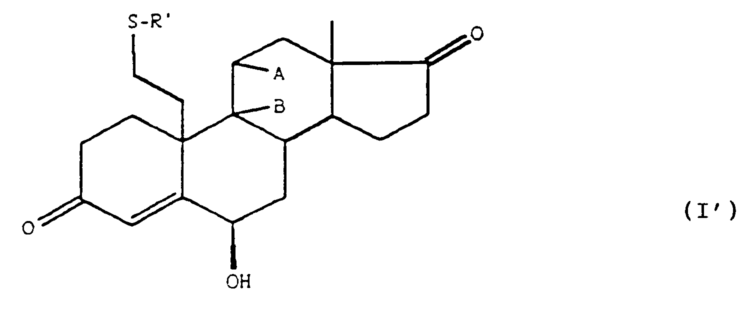 in which A and B have the previous meaning and R' represents an alkyl radical having at most 4 carbon atoms characterized in that a product in which R represents an alkyl radical having at most 4 carbon atoms is used as a reactive derivative which is reacted on the product of formula (IVₐ) or (IV_{b}) or as a salt of formula RSA which is reacted on the product of formula (IVₐ) or (IV_{b}).

## Claims (Claims for the following Contracting State(s): GR)

1. Preparation process for the products of general formula (I): 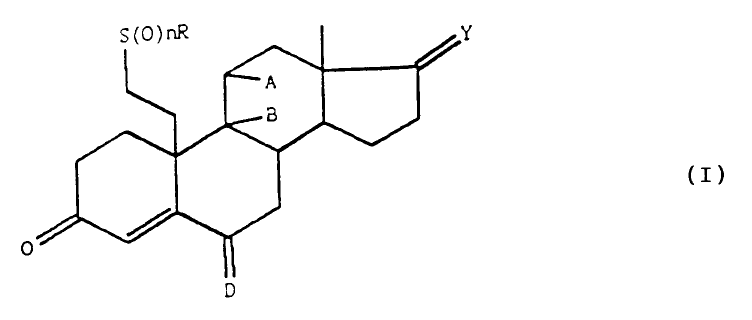 in which the substituent R represents an alkyl, alkenyl or alkynyl radical having at most 4 carbon atoms, Y represents an oxygen atom or a remainder: 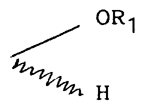 in which R₁ represents a hydrogen atom or an acyl radical,
n represents an integer from 0 to 2,
D represents an oxygen atom or a remainder 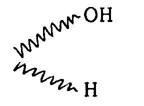 A and B together form an alpha epoxide function or with the carbon atoms to which they are linked, a second bond between these carbons, characterized in that a product of formula (II): 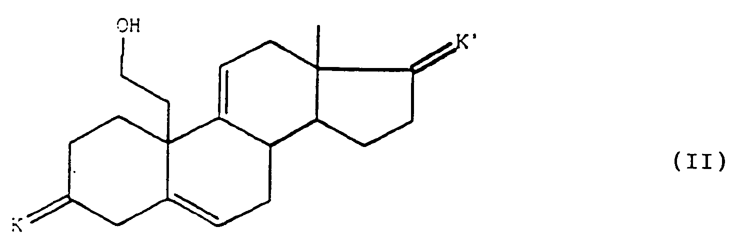 in which K and K', identical or different, represent a protected keto radical, is subjected to the action of an epoxidation reagent in order to obtain the products of formula (IIIa) and (IIIb): 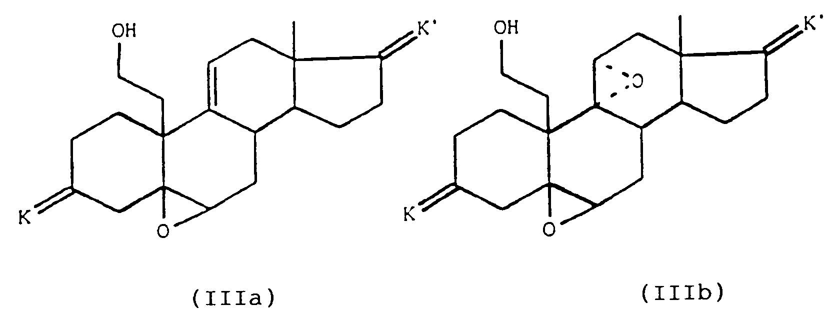 which products are treated with a conversion reagent of the hydroxy radical into a thiol radical in order to obtain the products of formulae (IVa) and (IVb): 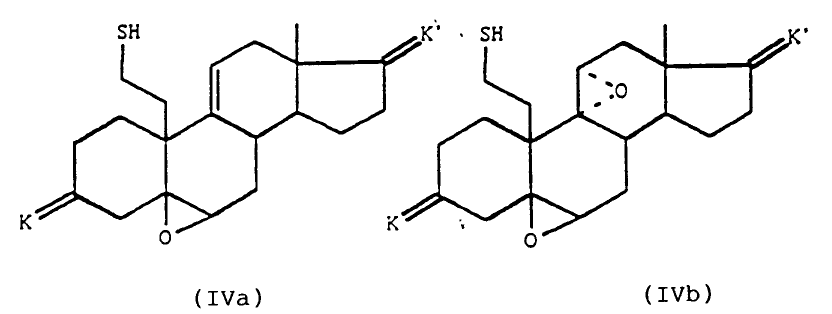 which are treated with a reactive derivative of the R radical, R having the meaning indicated above, in order to obtain the products of formulae (Va) and (Vb): 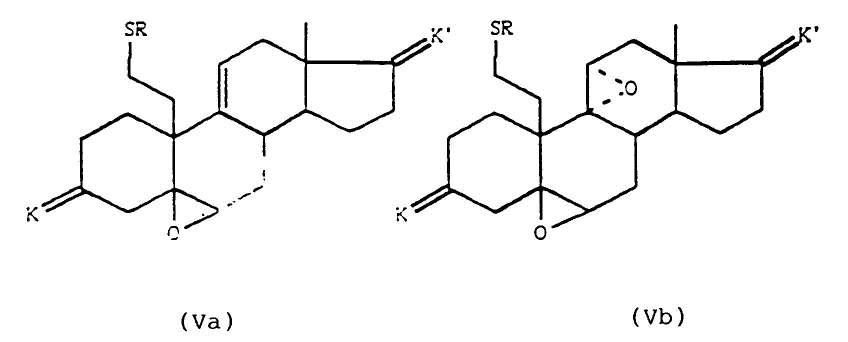 or with a reactive derivative of a sulphonic acid in order to obtain the products of formulae (VIa) and (VIb): 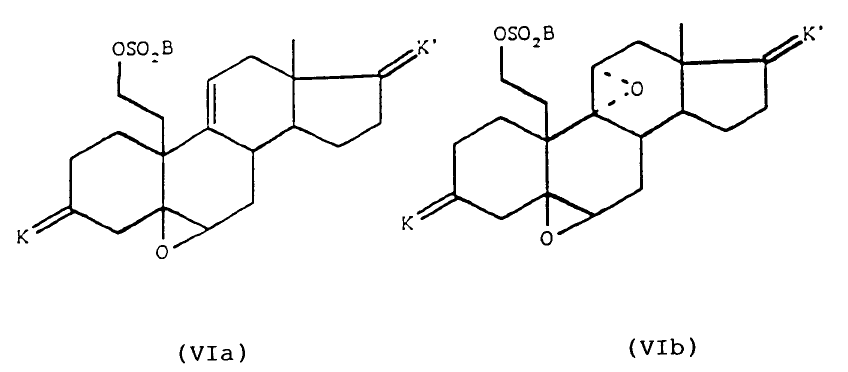 in which B represents the remainder of a sulphonic acid, which products of formulae (VIa) and (VIb) are treated with a salt of formula RSA in which A represents a monovalent cation in order to obtain products of formulae (Va) and (Vb) as defined above which products of formulae (Va) and (Vb) are treated with a deprotection reagent of the protected ketone functions and opening of the epoxide in position 5(6) in order to obtain the products of formulae (I'a) and (I'b): 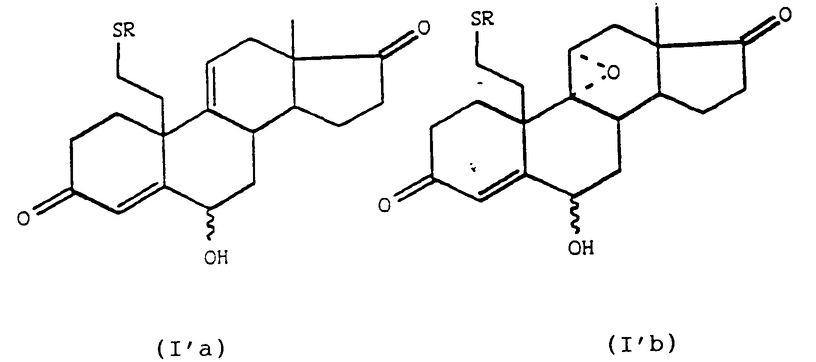 corresponding to products of formula (I) in which R has the meanings indicated in claim 1, Y represents an oxygen atom, n represents the number 0, and D represents a remainder: 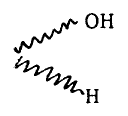 which products of formula (I'a) and (I'b) are, if desired, subjected to any one of the following reactions:
a) reduction optionally followed by an acylation of the ketone function in position 17,
b) oxidation of the sulphur atom of the -SR radical into the sulphoxide or sulphone,
c) oxidation of the hydroxyl function in position 6 into the ketone.

2. Process according to claim 1, characterized in that the conversion of the products of formulae (Va) and (Vb) is carried out under carefully-controlled conditions in order to obtain the products of formulae (VIIa) and (VIIb): 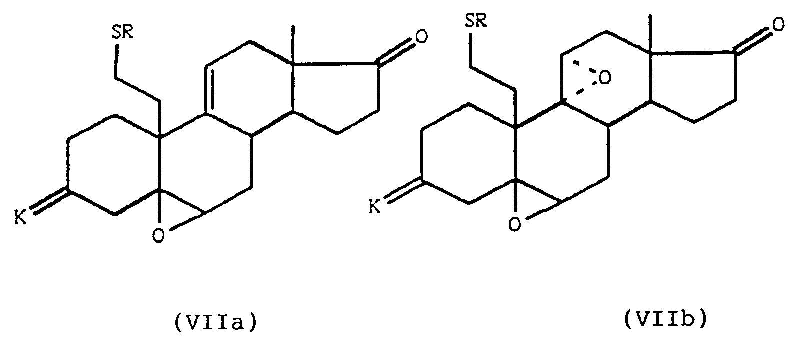 which products of formulae (VIIa) and (VIIb) are treated with a deprotection reagent of the ketone function and opening of the epoxide in position 5(6) in order to obtain the products of formulae (I'a) and (I'b) as defined in claim 1.

3. Process according to claim 1 or 2 for the preparation of products of formula (I) corresponding to general formula (I'): 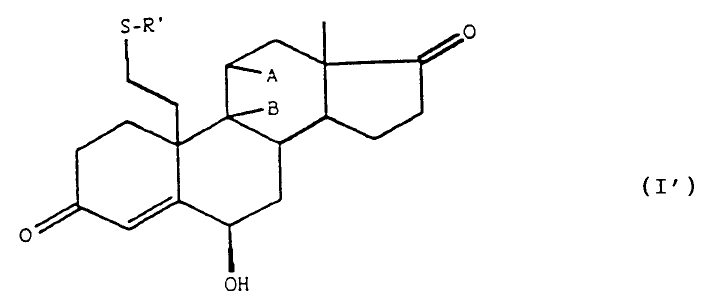 in which A and B have the previous meaning and R' represents an alkyl radical having at most 4 carbon atoms characterized in that a product in which R represents an alkyl radical having at most 4 carbon atoms is used as reactive derivative which is reacted on the product of formula (IVₐ) or (IV_{b}) or as a salt of formula RSA which is reacted on the product of formula (IVₐ) or (IV_{b}) .

4. Process according to any one of claims 1 to 3, characterized in that the reagents are chosen such that one of the products corresponding to the following formulae is prepared:
- 6beta-hydroxy-10beta-[2-(methylthio)-ethyl]-estra-4,9(11)-diene-3,17-dione,
- 9alpha,11alpha-epoxy-6beta-hydroxy-10beta-[2-(methylthio)-ethyl]-estr-4-ene-3,17-dione.

5. Preparation process for pharmaceutical compositions, characterized in that at least one of the products of general formula (I) as defined in claim 1 is used as active ingredient in a form intended for this use.

6. Preparation process for pharmaceutical compositions, characterized in that at least one of the products of general formula (I) as defined in one of claims 2 to 4 is used as active ingredient in a form intended for this use.

7. As new industrial products, the products of general formulae (IIIa), (IIIb), (IVa), (IVb), (Va), (Vb), (VIₐ) and (VI_{b}) as defined in claim 1 and the products of general formulae (VIIa) and (VIIb) as defined in claim 2.

## Patentansprüche (Patentansprüche für folgende(n) Vertragsstaat(en): AT, BE, CH, DE, DK, FR, GB, IT, LI, LU, NL, PT, SE)

1. Produkte der allgemeinen Formel (I) 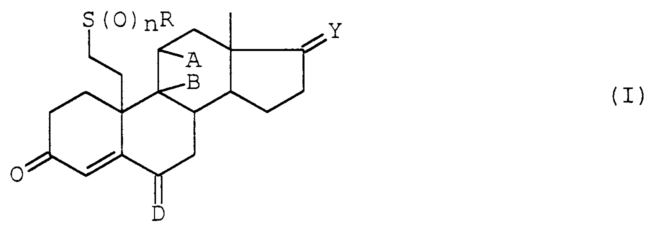 worin der Substituent R einen Alkyl-, Alkenyl- oder Alkinylrest mit höchstens 4 Kohlenstoffatomen darstellt,
Y ein Sauerstoffatom oder einen Rest 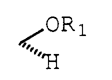 bedeutet, worin R₁ ein Wasserstoffatom oder ein Acylrest ist,
n eine ganze Zahl von 0 bis 2 ist,
D ein Sauerstoffatom oder einen Rest 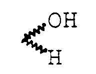 darstellt,
A und B zusammen eine α-Epoxyd-Funktion oder mit den Kohlenstoffatomen, an die sie gebunden sind, eine zweite Bindung zwischen diesen Kohlenstoffen bilden.

2. Produkte der allgemeinen Formel (I') 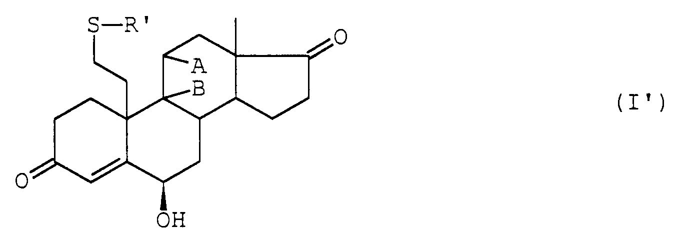 in der A und B die vorstehende Bedeutung besitzen und R' einen Alkylrest mit höchstens 4 Kohlenstoffatomen darstellt.

3. Produkte der allgemeinen Formel (I') wie in Anspruch 2 definiert, die den folgenden Formeln entsprechen:
- 6β-Hydroxy-10β-[2-(methylthio)-ethyl]-estra-4,9(11)-dien-3,17-dion,
- 9α,11α-Epoxy-6β-hydroxy-10β-[2-(methylthio)-ethyl]-estr-4-en-3,17-dion.

4. Verfahren zur Herstellung der Produkte der allgemeinen Formel (I) wie in Anspruch 1 definiert, dadurch gekennzeichnet, daß man ein Produkt der Formel (II) 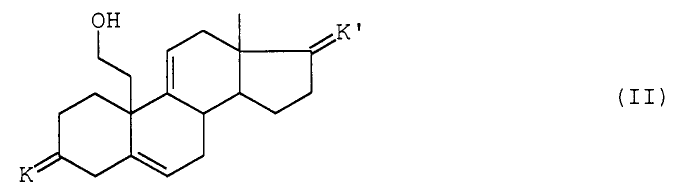 in der K und K', gleich oder verschieden, einen geschützten Ketorest darstellen, der Einwirkung eines Epoxydierungs-Reaktanden unterzieht, um die Produkte der Formeln (IIIa) und (IIIb) 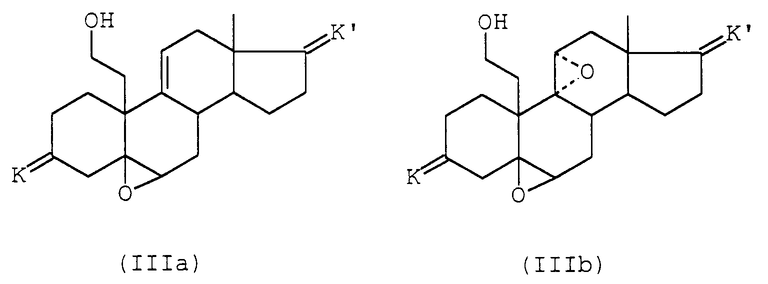 zu erhalten, die man mit einem Reaktanden zur Umwandlung des Hydroxyrestes in einen Thiorest behandelt, um die Produkte der Formeln (IVa) und (IVb) 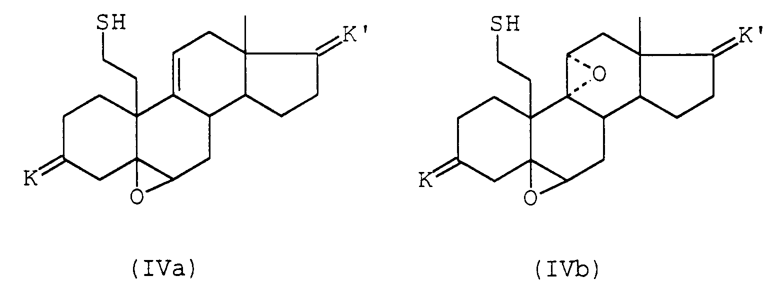 zu erhalten, die man mit einem reaktiven Derivat des Restes R behandelt, wobei R die in Anspruch 1 angegebene Bedeutung besitzt, um die Produkte der Formeln (Va) und (Vb) 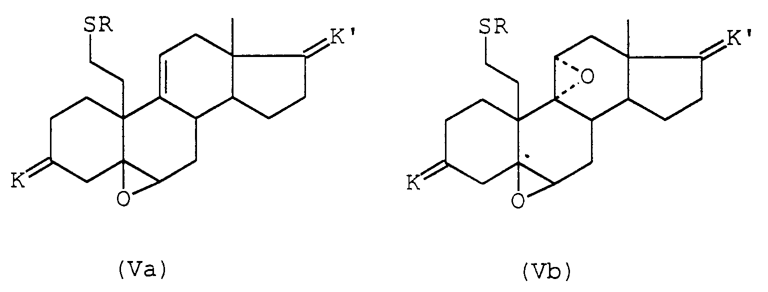 zu erhalten, die man mit einem Reaktanden zur Abspaltung der Schutzgruppe von den geschützten Ketonfunktionen und zur Öffnung des Epoxydes in Position 5(6) behandelt, um die Produkte der Formeln (I'a) und (I'b) 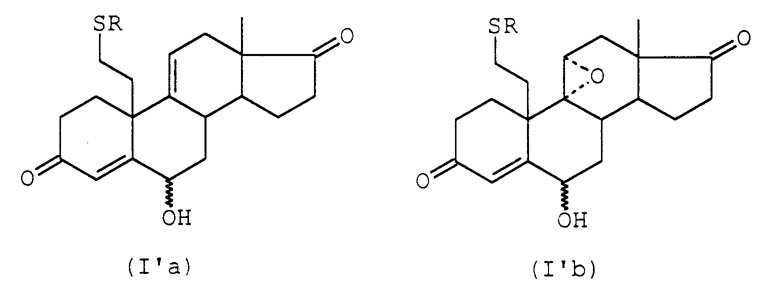 zu erhalten, die den Produkten der Formel (I) entsprechen, in der R die in Anspruch 1 angegebene Bedeutung besitzt, Y ein Sauerstoffatom darstellt, n die Zahl 0 ist und D einen Rest 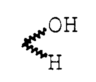 bedeutet, wobei die Produkte der Formeln (I'a) und (I'b), wenn erwünscht, irgendeiner der folgenden Reaktionen unterzogen werden:
a) Reduktion, gegebenenfalls gefolgt von einer Acylierung der Ketonfunktion in Position 17,
b) Oxidation des Schwefelatoms des Restes -SR zum Sulfoxid oder Sulfon,
c) Oxidation der Hydroxylfunktion in Position 6 zum Keton.

5. Verfahren zur Herstellung der Produkte der allgemeinen Formel (I) wie in Anspruch 1 definiert, dadurch gekennzeichnet, daß man ein Produkt der Formel (II) 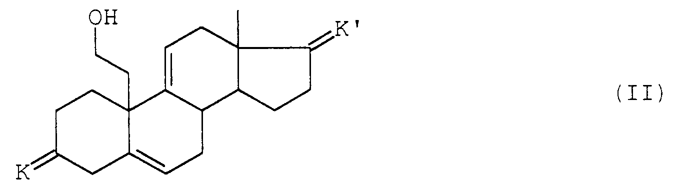 in der K und K', gleich oder verschieden, einen geschützten Ketorest darstellen, der Einwirkung eines Epoxydierungs-Reaktanden unterzieht, um die Produkte der Formeln (IIIa) und (IIIb) 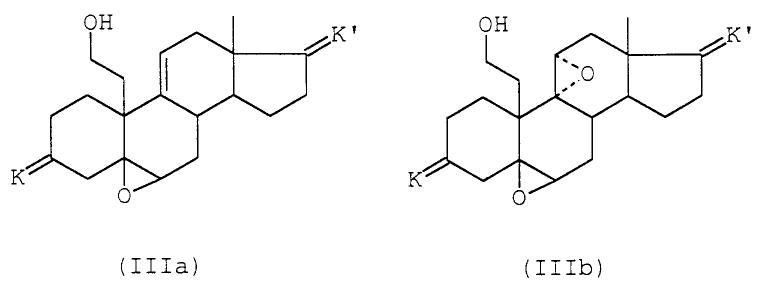 zu erhalten, die man mit einem reaktiven Derivat einer Sulfonsäure behandelt, um die Produkte der Formeln (VIa) und (VIb) 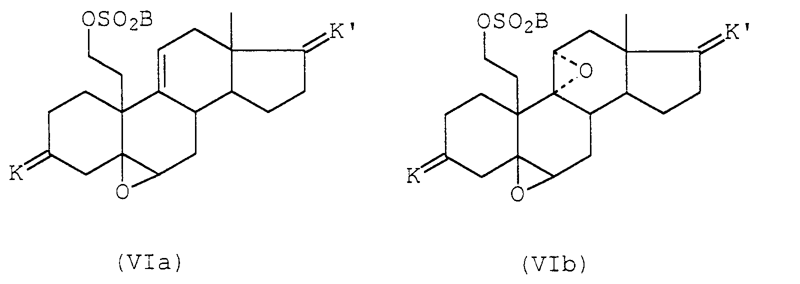 zu erhalten, worin B den Rest einer Sulfonsäure darstellt, und man die Produkte der Formeln (VIa) und (VIb) mit einem Salz der Formel RSA behandelt, in der A ein monovalentes Kation darstellt und R die in Anspruch 1 angegebene Bedeutung besitzt, um die Produkte der Formeln (Va) und (Vb) 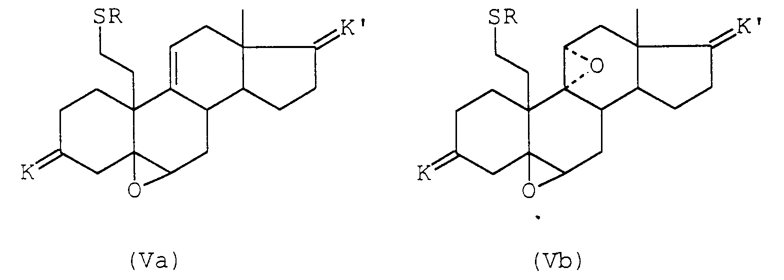 zu erhalten, die man mit einem Reaktanden zur Abspaltung der Schutzgruppen von den geschützten Ketonfunktionen und zur Öffnung des Epoxydes in Position 5(6) behandelt, um die Produkte der Formeln (I'a) und (I'b) 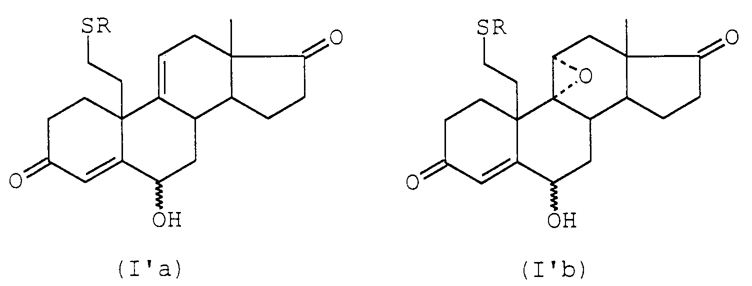 zu erhalten, die den Produkten der Formel (I) entsprechen, in der R die in Anspruch 1 angegebene Bedeutung besitzt, Y ein Sauerstoffatom darstellt, n die Zahl 0 ist und D einen Rest 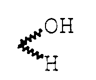 bedeutet, wobei die Produkte der Formeln (I'a) und (I'b), wenn erwünscht, irgendeiner der folgenden Reaktionen unterzogen werden:
a) Reduktion, gegebenenfalls gefolgt von einer Acylierung der Ketonfunktion in Position 17,
b) Oxidation des Schwefelatoms des Restes -SR zum Sulfoxid oder Sulfon,
c) Oxidation der Hydroxylfunktion in Position 6 zum Keton.

6. Verfahren nach einem der Ansprüche 4 und 5, dadurch gekennzeichnet, daß die Umwandlung der Produkte der Formeln (Va) und (Vb) unter schonenden Bedingungen durchgeführt wird, um die Produkte der Formeln (VIIa) und (VIIb) 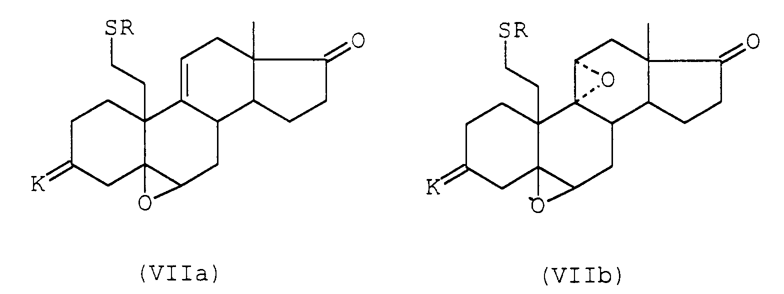 zu erhalten, die man mit einem Reaktanden zur Abspaltung der Schutzgruppe von der geschützten Ketonfunktion und zur Öffnung des Epoxydes in Position 5(6) behandelt, um die wie in Anspruch 5 definierten Produkte der Formeln (I'a) und (I'b) zu erhalten.

7. Als Medikamente die Produkte der allgemeinen Formel (I) wie in Anspruch 1 definiert.

8. Als Medikamente die Produkte der allgemeinen Formel (I') wie in einem der Ansprüche 2 oder 3 definiert.

9. Pharmazeutische Zusammensetzungen, die als Wirkstoff mindestens eines der in einem der Ansprüche 7 oder 8 definierten Medikamente enthalten.

10. Als neue industrielle Produkte die Produkte der allgemeinen Formeln (IIIa), (IIIb), (IVa), (IVb), (Va), (Vb), wie in Anspruch 4 definiert, die Produkte der allgemeinen Formeln (VIa), (VIb), wie in Anspruch 5 definiert, und die Produkte der allgemeinen Formeln (VIIa), (VIIb), wie in Anspruch 6 definiert.

## Patentansprüche (Patentansprüche für folgende(n) Vertragsstaat(en): ES)

1. Verfahren zur Herstellung von Produkten der allgemeinen Formel (I) 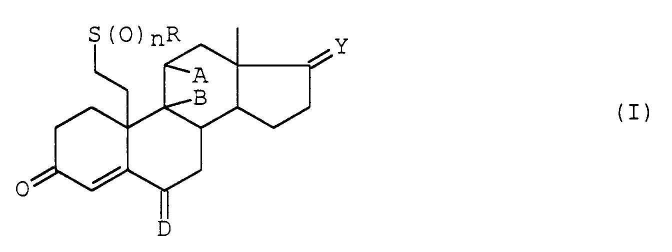 worin der Substituent R einen Alkyl-, Alkenyl- oder Alkinylrest mit höchstens 4 Kohlenstoffatomen darstellt,
Y ein Sauerstoffatom oder einen Rest 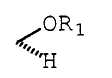 bedeutet, worin R₁ ein Wasserstoffatom oder ein Acylrest ist,
n eine ganze Zahl von 0 bis 2 ist,
D ein Sauerstoffatom oder einen Rest 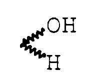 darstellt,
A und B zusammen eine α-Epoxyd-Funktion oder mit den Kohlenstoffatomen, an die sie gebunden sind, eine zweite Bindung zwischen diesen Kohlenstoffen bilden, dadurch gekennzeichnet, daß man ein Produkt der Formel (II) 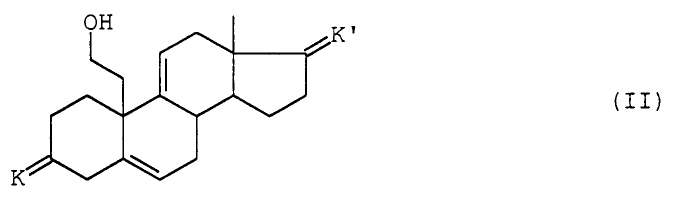 in der K und K', gleich oder verschieden, einen geschützten Ketorest darstellen, der Einwirkung eines Epoxydierungs-Reaktanden unterzieht, um die Produkte der Formeln (IIIa) und (IIIb) 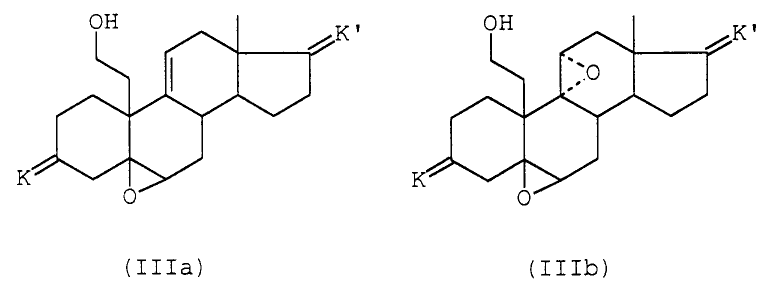 zu erhalten, die man mit einem Reaktanden zur Umwandlung des Hydroxyrestes in einen Thiorest behandelt, um die Produkte der Formeln (IVa) und (IVb) 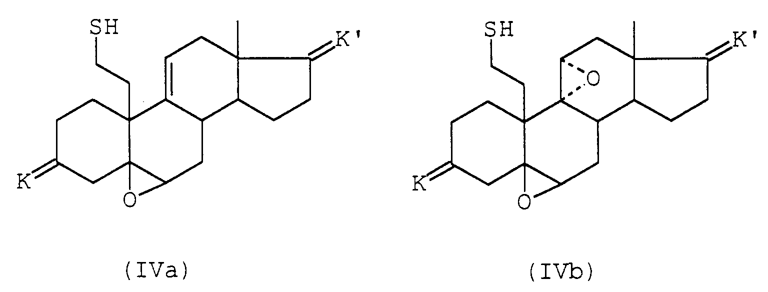 zu erhalten, die man mit einem reaktiven Derivat des Restes R behandelt, wobei R die oben angegebene Bedeutung besitzt, um die Produkte der Formeln (Va) und (Vb) 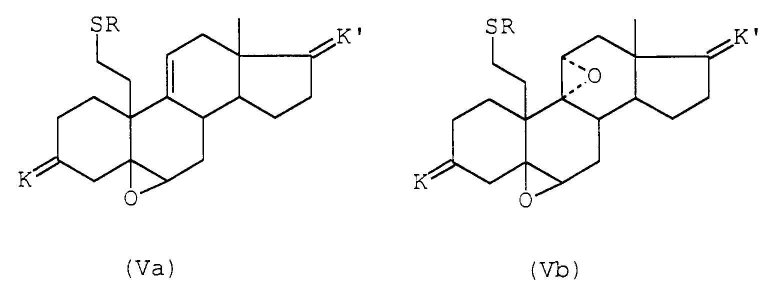 zu erhalten,
oder die man mit einem reaktiven Derivat einer Sulfonsäure behandelt, um die Produkte der Formeln (VIa) und (VIb) 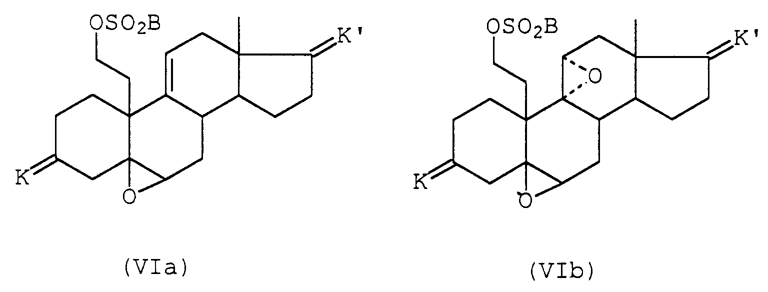 zu erhalten, worin B den Rest einer Sulfonsäure darstellt, und man die Produkte der Formeln (VIa) und (VIb) mit einem Salz der Formel RSA behandelt, in der A ein monovalentes Kation darstellt, um die wie oben definierten Produkte der Formeln (Va) und (Vb) zu erhalten, die man mit einem Reaktanden zur Abspaltung der Schutzgruppen von den geschützten Ketonfunktionen und zur Öffnung des Epoxydes in Position 5(6) behandelt, um die Produkte der Formeln (I'a) und (I'b) 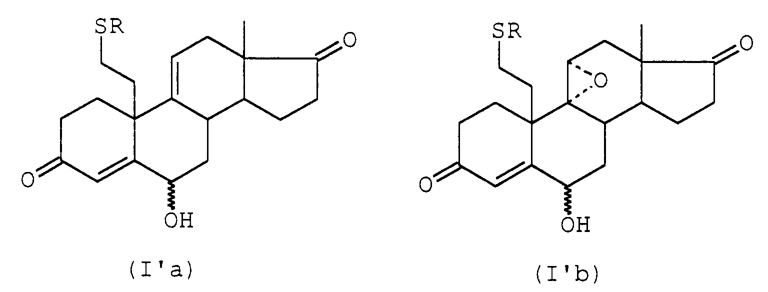 zu erhalten, die den Produkten der Formel (I) entsprechen, in der R die oben angegebene Bedeutung besitzt, Y ein Sauerstoffatom darstellt, n die Zahl 0 ist und D einen Rest 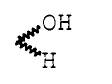 bedeutet, wobei die Produkte der Formeln (I'a) und (I'b), wenn erwünscht, irgendeiner der folgenden Reaktionen unterzogen werden:
a) Reduktion, gegebenenfalls gefolgt von einer Acylierung der Ketonfunktion in Position 17,
b) Oxidation des Schwefelatoms des Restes -SR zum Sulfoxid oder Sulfon,
c) Oxidation der Hydroxylfunktion in Position 6 zum Keton.

2. Verfahren nach Anspruch 1, dadurch gekennzeichnet, daß die Umwandlung der Produkte der Formeln (Va) und (Vb) unter schonenden Bedingungen durchgeführt wird, um die Produkte der Formeln (VIIa) und (VIIb) 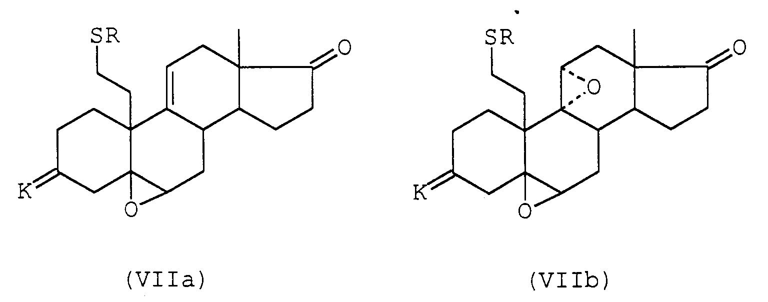 zu erhalten, die man mit einem Reaktanden zur Abspaltung der Schutzgruppe von der geschützten Ketonfunktion und zur Öffnung des Epoxydes in Position 5(6) behandelt, um die wie in Anspruch 1 definierten Produkte der Formeln (I'a) und (I'b) zu erhalten.

3. Verfahren nach Anspruch 1 oder 2 zur Herstellung der Produkte der allgemeinen Formel (I') 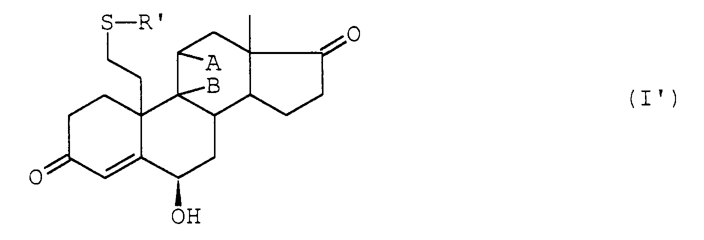 in der A und B die vorstehende Bedeutung besitzen und R' einen Alkylrest mit höchstens 4 Kohlenstoffatomen darstellt, dadurch gekennzeichnet, daß man als reaktives Derivat, das man mit dem Produkt der Formel (IVa) oder (IVb), oder als Salz der Formel RSA, das man mit dem Produkt der Formel (IVa) oder (IVb) zur Reaktion bringt, ein Produkt verwendet, worin R einen Alkylrest mit höchstens 4 Kohlenstoffatomen darstellt.

## Patentansprüche (Patentansprüche für folgende(n) Vertragsstaat(en): GR)

1. Verfahren zur Herstellung von Produkten der allgemeinen Formel (I) 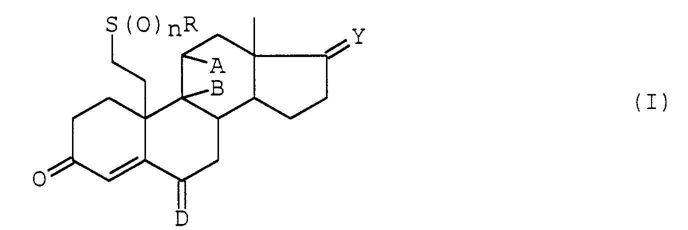 worin der Substituent R einen Alkyl-, Alkenyl- oder Alkinylrest mit höchstens 4 Kohlenstoffatomen darstellt,
Y ein Sauerstoffatom oder einen Rest 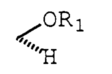 bedeutet, worin R₁ ein Wasserstoffatom oder ein Acylrest ist,
n eine ganze Zahl von 0 bis 2 ist,
D ein Sauerstoffatom oder einen Rest 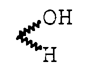 darstellt,
A und B zusammen eine α-Epoxyd-Funktion oder mit den Kohlenstoffatomen, an die sie gebunden sind, eine zweite Bindung zwischen diesen Kohlenstoffen bilden, dadurch gekennzeichnet, daß man ein Produkt der Formel (II) 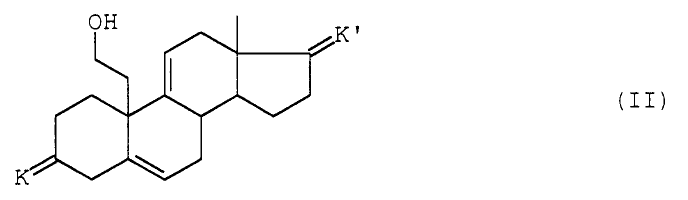 in der K und K', gleich oder verschieden, einen geschützten Ketorest darstellen, der Einwirkung eines Epoxydierungs-Reaktanden unterzieht, um die Produkte der Formeln (IIIa) und (IIIb) 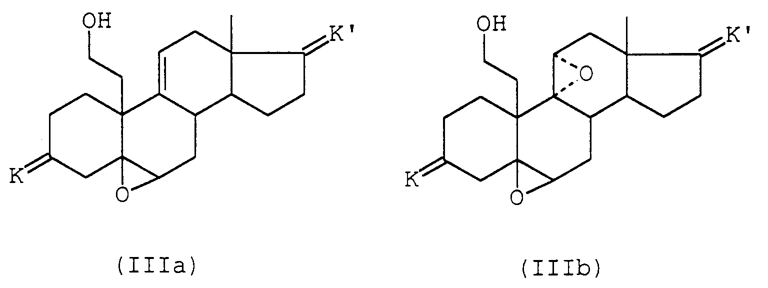 zu erhalten, die man mit einem Reaktanden zur Umwandlung des Hydroxyrestes in einen Thiorest behandelt, um die Produkte der Formeln (IVa) und (IVb) 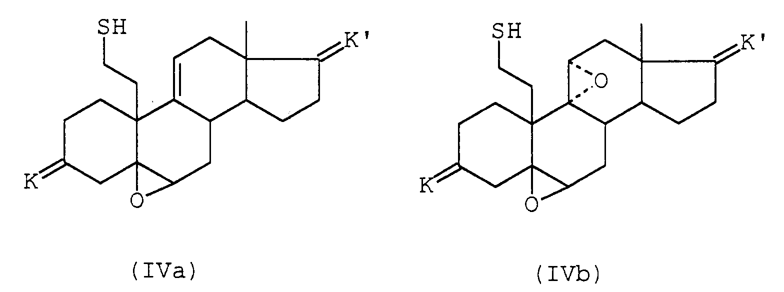 zu erhalten, die man mit einem reaktiven Derivat des Restes R behandelt, wobei R die oben angegebene Bedeutung besitzt, um die Produkte der Formeln (Va) und (Vb) 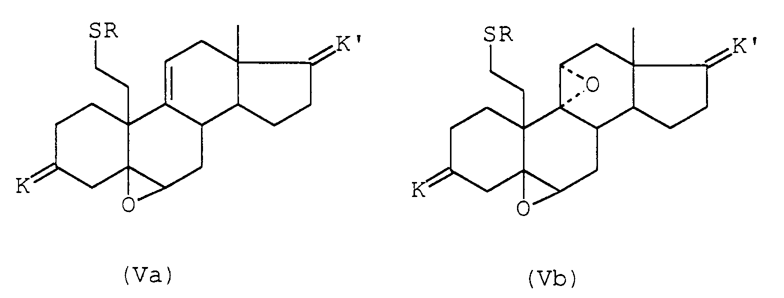 zu erhalten,
oder die man mit einem reaktiven Derivat einer Sulfonsäure behandelt, um die Produkte der Formeln (VIa) und (VIb) 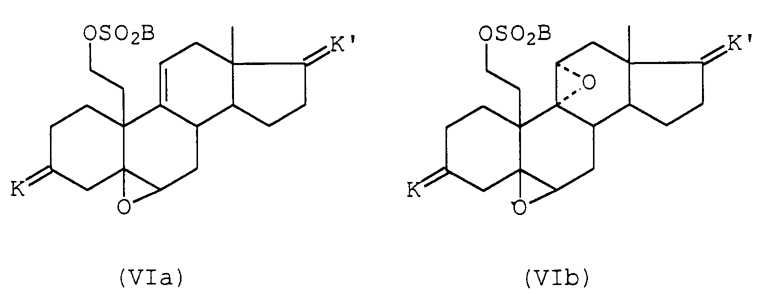 zu erhalten, worin B den Rest einer Sulfonsäure darstellt, und man die Produkte der Formeln (VIa) und (VIb) mit einem Salz der Formel RSA behandelt, in der A ein monovalentes Kation darstellt, um die wie oben definierten Produkte der Formeln (Va) und (Vb) zu erhalten, die man mit einem Reaktanden zur Abspaltung der Schutzgruppen von den geschützten Ketonfunktionen und zur Öffnung des Epoxydes in Position 5(6) behandelt, um die Produkte der Formeln (I'a) und (I'b) 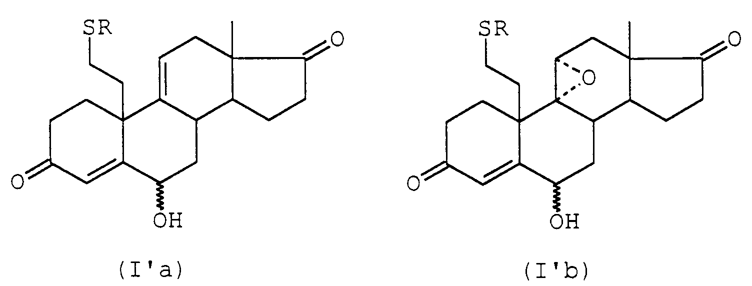 zu erhalten, die den Produkten der Formel (I) entsprechen, in der R die oben angegebene Bedeutung besitzt, Y ein Sauerstoffatom darstellt, n die Zahl 0 ist und D einen Rest 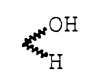 bedeutet, wobei die Produkte der Formeln (I'a) und (I'b), wenn erwünscht, irgendeiner der folgenden Reaktionen unterzogen werden:
a) Reduktion, gegebenenfalls gefolgt von einer Acylierung der Ketonfunktion in Position 17,
b) Oxidation des Schwefelatoms des Restes -SR zum Sulfoxid oder Sulfon,
c) Oxidation der Hydroxylfunktion in Position 6 zum Keton.

2. Verfahren nach Anspruch 1, dadurch gekennzeichnet, daß die Umwandlung der Produkte der Formeln (Va) und (Vb) unter schonenden Bedingungen durchgeführt wird, um die Produkte der Formeln (VIIa) und (VIIb) 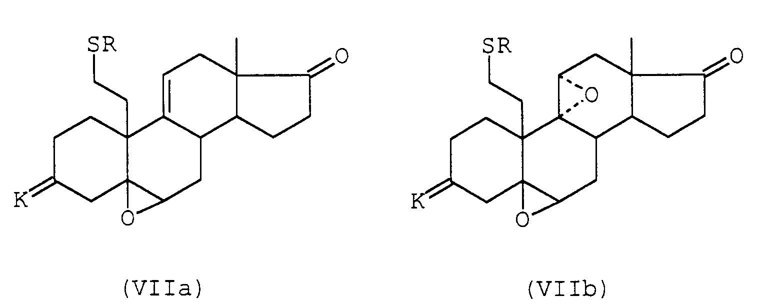 zu erhalten, die man mit einem Reaktanden zur Abspaltung der Schutzgruppe von der geschützten Ketonfunktion und zur Öffnung des Epoxydes in Position 5(6) behandelt, um die wie in Anspruch 1 definierten Produkte der Formeln (I'a) und (I'b) zu erhalten.

3. Verfahren nach Anspruch 1 oder 2 zur Herstellung der Produkte der allgemeinen Formel (I') 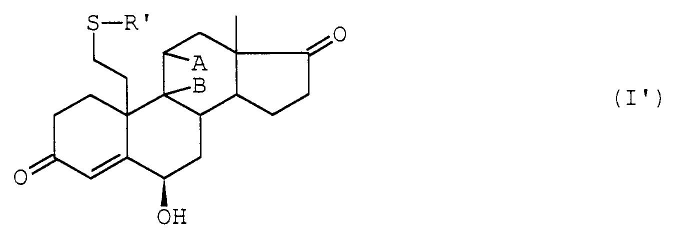 in der A und B die vorstehende Bedeutung besitzen und R' einen Alkylrest mit höchstens 4 Kohlenstoffatomen darstellt, dadurch gekennzeichnet, daß man als reaktives Derivat, das man mit dem Produkt der Formel (IVa) oder (IVb), oder als Salz der Formel RSA, das man mit dem Produkt der Formel (IVa) oder (IVb) zur Reaktion bringt, ein Produkt verwendet, worin R einen Alkylrest mit höchstens 4 Kohlenstoffatomen darstellt.

4. Verfahren nach irgendeinem der Ansprüche 1 bis 3, dadurch gekennzeichnet, daß man die Reaktanden in der Weise auswählt, daß man eines der Produkte herstellt, die den folgenden Formeln entsprechen:
- 6β-Hydroxy-10β-[2-(methylthio)-ethyl]-estra-4,9(11)-dien-3,17-dion,
- 9α,11α-Epoxy-6β-hydroxy-10β-[2-(methylthio)-ethyl]-estr-4-en-3,17-dion.

5. Verfahren zur Herstellung von pharmazeutischen Zusammensetzungen, dadurch gekennzeichnet, daß man als Wirkstoff mindestens eines der Produkte der allgemeinen Formel (I), wie in Anspruch 1 definiert, in eine für diese Anwendung vorgesehene Form bringt.

6. Verfahren zur Herstellung von pharmazeutischen Zusammensetzungen, dadurch gekennzeichnet, daß man als Wirkstoff mindestens eines der Produkte der allgemeinen Formel (I), wie in einem der Ansprüche 2 bis 4 definiert, in eine für diese Anwendung vorgesehene Form bringt.

7. Als neue industrielle Produkte die Produkte der allgemeinen Formeln (IIIa), (IIIb), (IVa), (IVb), (Va), (Vb), (VIa) und (VIb), wie in Anspruch 1 definiert, und die Produkte der allgemeinen Formeln (VIIa), (VIIb), wie in Anspruch 2 definiert.
